(19) Europäisches Patentamt
European Patent Office
Office européen des brevets

(11) EP 2 942 622 B1

(12) **FASCICULE DE BREVET EUROPEEN**

(45) Date de publication et mention de la délivrance du brevet:
**06.07.2022 Bulletin 2022/27**

(21) Numéro de dépôt: **15290117.9**

(22) Date de dépôt: **29.04.2015**

(51) Classification Internationale des Brevets (IPC):
***G01N 33/24** (2006.01)* ***G01N 33/00** (2006.01)*

(52) Classification Coopérative des Brevets (CPC):
**G01N 33/0098**

(54) **MÉTHODE DE DÉTERMINATION DE TENEURS CRITIQUES EN AZOTE DE CULTURES**

BESTIMMUNGSMETHODE DES KRITISCHEN GEHALTS VON STICKSTOFF IN PFLANZENKULTUREN

METHOD FOR DETERMINING CRITICAL NITROGEN CONTENTS OF CROPS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.05.2014 FR 1401032**

(43) Date de publication de la demande:
**11.11.2015 Bulletin 2015/46**

(73) Titulaire: **Polyor SARL**
**54000 Nancy (FR)**

(72) Inventeur: **Claude, Pierre-Philippe**
**54000 Nancy (FR)**

(56) Documents cités:
**EP-A1- 2 671 443**

- **LEMAIRE ET AL: "Diagnosis tool for plant and crop N status in vegetative stage", EUROPEAN JOURNAL OF AGRONOMY, ELSEVIER, AMSTERDAM, NL, vol. 28, no. 4, 11 mars 2008 (2008-03-11), pages 614-624, XP022575272, ISSN: 1161-0301**
- **LEMAIRE ET AL: "Is crop N demand more closely related to dry matter accumulation or leaf area expansion during vegetative growth?", FIELD CROPS RESEARCH, ELSEVIER, AMSTERDAM, NL, vol. 100, no. 1, 4 janvier 2007 (2007-01-04), pages 91-106, XP005707819, ISSN: 0378-4290, DOI: 10.1016/J.FCR.2006.05.009**
- **YAO XIA ET AL: "Development of critical nitrogen dilution curve in rice based on leaf dry matter", EUROPEAN JOURNAL OF AGRONOMY, ELSEVIER, AMSTERDAM, NL, vol. 55, 22 January 2014 (2014-01-22), pages 20-28, XP028636345, ISSN: 1161-0301, DOI: 10.1016/J.EJA.2013.12.004**
- **Xia Yao ET AL: "Using leaf dry matter to quantify the critical nitrogen dilution curve for winter wheat cultivated in eastern China", Field Crops Research, vol. 159, 1 March 2014 (2014-03-01), pages 33-42, XP055693296, AMSTERDAM, NL ISSN: 0378-4290, DOI: 10.1016/j.fcr.2013.12.007**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

## DOMAINE TECHNIQUE DE L'INVENTION

Agronomie, suivi de l'état nutritionnel des cultures, dit au pilotage de la fertilisation, et plus particulièrement expérimentation agronomique, *in situ* notamment. Il est aussi question d'informatique et de modélisation dynamique de processus sol/plante, notamment en présence de grandes cultures agronomiques.

## ÉTAT DE LA TECHNIQUE

L'expérimentation agronomique *in situ* est balbutiante. Elle implique généralement à ce stade l'alignement côtes à côtes des bandes de cultures agronomiques traitées et témoins (non traitées).

Au pire les moyennes de quelques prélèvements analysés provenant de chaque bande, est comparée ; au mieux, il s'agit d'Anova putatives supposant que de tels dispositifs en bandes sont défendable entant que tels *(eg.* Bermudez et Mallarino 2002, 2004), ce qui est rarement le cas.

D'une manière ou d'une autre, il faut dans ces cas là estimer les biomasses et/ou rendements en grains par hectare à partir de tels prélèvements immanquablement imprécis du fait de leurs superficies limitées.

Pourtant, la variabilité inter - plants et/ou intra - parcellaire est importante. Bien que problématique d'un point de vue de la production agronomique de rendements récoltables (*eg*. FR 2 910 230), elle peut, en principe, être exploitée lors d'expérimentation agronomiques *in situ.* En effet, il est possible, en principe, d'établir des corrélations entre les *poids spécifiques* (PS ; g/plant) de plants récoltés individuellement et certaines variables phytométriques telles que la teneur élémentaire des parties aériennes du plant, le rendement quantique maximal (Fv/Fm), l'indice de nutrition azoté (NNI ; Juste et al. 1994) et/ou un quelconque indice phyto-pathométrique (iPathos ; EP 2 572 581) du feuillage de la culture agronomique en cours.

Or, du fait de l'importance de l'azote en production végétale, dans la pratique c'est la corrélation entre de tels indices et l'NNI qui est la plus révélatrice d'éventuels effets sur les plants traités par rapport aux plants témoins non traités. Cela suppose d'avoir préalablement établi NNI en fonction de PS, ce qui n'est pas du tout le cas aujourd'hui, le courbes de dilution critique de l'N proposées par Justes et al. 1994 nécessitant la détermination directe de la matière sèches des parties aériennes (MSPA) par unités de surface arable, généralement en tonne par hectare (t/ha) comme illustré dans Yao et al. 2014. Or, cette détermination est fastidieuse dans la pratique agronomique et source d'imprécisions ; elle n'est donc pas véritablement mise en oeuvre lors de la détermination routinière ou même expérimentale d'NNI. En effet, les outils de pilotage de la fertilisation azotée, qui pourtant s'appuient implicitement sur ce concept d'NNI, ne mesurent pas dans les faits directement NNI, mais au lieu certains indices des teneurs en N supposément et empiriquement corrélés avec NNI ; *eg*. chlorophylle (SPAD-502™, Konica-Minolta, Japon), azote foliaire (N-Testeur™, Yara France), nitrates des sèves (Jubil™, Challenge Agriculture, France), composés polyphénoliques (Force-A™, Orsay, France, *cf.* Tremblay et al. 2009), l'NDVI (Normalized Différence Végétation Index), etc.

Prédire ponctuellement sur une parcelle agricole la corrélation entre le poids spécifique (PS, en g de matière sèche par plantule) serait donc utile lors d'expérimentations agronomiques. Cela permettra d'effectuer des essais particuliers chez l'agriculteur, *in situ* en sorte, sans avoir à installer de coûteux dispositifs expérimentaux et/ou estimer de manière très imprécise les MSPA par hectare afin de déterminer NNI selon Justes et al. 1994. On notera en ce sens que le PS est aussi intrinsèquement une mesure facile à obtenir et, surtout, bien plus précise qu'une quelconque dérivation de la MSPA par ha d'échantillons superficiels nécessairement imprécis.

Les PS dépendent de la densité locale des peuplements imposée à une plantule individuelle. Il s'agit donc de relations *allométriques* (Deng *et al.* 2012, 2012 bis) entre PS et une quelconque variable agronomique et/ou phytométrique. Ces variables sont avantageusement des ratios entre les valeurs observées pour la plantule de PS donné, et les valeurs critiques ou seuils fonctions de PS. Or, ces fonctions comportant le PS ne sont pas connues empiriquement.

Définitions

**MSPA** : matières sèche des parties aériennes d'une culture, généralement exprimée en tonnes par hectare (t/ha)
**Nc** (ou Nct parfois) : teneur critique en azote (% MSPA) de la MSPA selon Justes et al. 1994 en dessous de la quelle la production de MSPA par la culture en cour est compromise
**Na** (ou simplement N selon le cas) : teneur en azote (% MSPA) de la MSPA actuellement (sic) mesurée.
**NNI** : indice de nutrition azotée, ou *nitrogen nutrition index* (NNI ; Justes et al. 1994), i.e. le ratio Na/Nc
**pNNI** : idem à NNI, sauf que Na et **Nc** sont déterminées sur la base du poids spécifique (PS ; g-MSPA/plant) plutôt que du poids hectoriel (PH ; t-MSPA/hectare)

**PH** : poids hectoriel de la MSPA en tonnes par hectare (t/ha)

**PS** : poids spécifique de la MSPA en g par plant (g/plant)

**P1, P2** : proportions (g/g) de la MSPA associées (P1) ou non (P2) aux processus de production de la biomasse, en particulier photosynthèse et nutrition minéral. Ainsi P2 = 1-**P1** n'intervient pas dans les processus de production de la biomasse.

**N1**, **N2** : teneurs en azote (g/g) de M1 et M2, respectivement

**M1**, **M2** : première et deuxième composantes de la MSPA associées (M1) ou non (M2) aux processus de production de la biomasse, en particulier photosynthèse et nutrition minéral. Ainsi, MSPA = M = M1 + M2.

**r(t)** : vitesse, ou taux, de croissance relative (g/semaine) d'un plant individuel

**n(t)** : teneur en azote (g-N/g-MSPA) d'un plant individuel

**k1** : coefficient de l'équation dM/dt = k1M1 qui est calculé à partir de la pente initiale des courbes de croissance de la MSPA (*eg.* ici Figures 3 et 4).

**a, b** : coefficients de l'équation n(t) = a $\times$ r(t) + b, avec a = (N1 - N2) /k1, et b = N2

## DIVULGATION DE L'INVENTION

*Problème technique*

L'expérimentation agronomique *in situ* est à ce jour très artisanale, et très souvent ne respecte pas les lois de probabilités ; elle se résume souvent à de simples comparaisons appariées répétées de 1 à 3 ou 4 fois. En ce sens, ces prélèvements ne servent que dans le cadre de campagne pré - commerciales devant générer de la sorte des douzaines, voire de centaines de telles comparaisons appariées. Or, les utilisateurs de nouveaux produits et services demandent à voir *in situ,* chez eux et sur leurs parcelles, et non simplement de participer de la sorte, à titre d'observateurs à un quelconque sondage pré-commercial.

Il s'agit d'un problème technique lié à l'expérimentation *in situ* lors d'essais agronomiques d'engrais et matières fertilisantes. Le nombre d'unités expérimentales étant réduit, pour des raisons de coûts, les agronomes en sont réduits à placer deux, voire trois bandes, généralement de la largeur de la rampe d'un pulvérisateur, et d'échantillonner de manière répétée ces bandes. On recherche ainsi à comparer les moyennes de ces répétitions à titre d'essais, chaque moyenne comportant ainsi au plus 3 à 4 observations. Pour des raisons évidentes, ce type de comparaisons, en termes d'inférence statistique, ne comporte aucune information et sont en soi, ponctuellement sur une parcelle agricole donnée, inutile. Pourtant, cette approche à l'expérimentation agronomique est la norme établie pour la plupart des essais technico-commerciaux dans le secteur des matières fertilisantes.

A priori, la solution technique consisterait à évaluer le poids spécifique (PS ; g/plant), et de transformer celui-ci en poids de biomasse par hectare (t/ha), et d'estimer ainsi l'indice de nutrition azoté (NNI - *nitrogen nutrition index* ; Justes et al. 1994) selon la masse par hectare (PH ; t/ha) calculé à partir de ces PS. Or, cette transformation est indirecte et imprécise. Il vaudra mieux convertir directement PS en NNI à l'aide d'une courbe de dilution critique de la teneur en N de la MSPA. Le cas échéant, il serait ainsi possible de comparer les courbes de réponse de teneurs élémentaires [e], e = N, P, K, Ca, Mg, *etc.,* ou encore de mesures fluométriques, phyto-pathométriques, *etc.,* à NNI. La comparaison de ces courbes à titre d'inférence statistique est, elle, statistiquement défendable.

Pour faire simple, il s'agit donc de préciser de telles courbes de dilution critique des teneurs en N des MSPA de plants individuels de PS donnés. Ces courbes Nc : PS n'apparaissent pas aujourd'hui dans la base de connaissance. Elles permettraient néanmoins de calculer à partir de simples PS un nouveau type NNI, disons "pNNI" pour faire la distinction, beaucoup plus facilement à obtenir, directement, qu'auparavant.

*Solution technique*

Pour rendre ce type d'expérimentation *in situ* sur la parcelle statistiquement défendable je propose d'exploiter la variabilité intra - parcellaire, voire inter - plants, sans recourir à une estimation des rendements agronomiques en grains ou de la biomasse. En effet, a priori ce type d'échantillonnage est imprécis et redondant s'il n'y a que deux ou trois bandes avec est sans le traitement côtes à côtes. Il faut plutôt déterminer le poids spécifique par plant ou plantule (PS ; g/plt) en un certain indice du potentiel azoto - nutritionnel ("pNNI") au lieu de l'habituel indice de nutrition azoté (NNI ; Juste et al. 1994) qui lui nécessite un estimé de la biomasse aérienne. Pour ce faire, il faut redéfinir la courbe de dilution critique de l'N, Nc, fonction de la MSPA superficielle en une courbe Nc fonction du poids spécifique (*eg.* g/plant) de la MSPA de plants individuels. Ce faisant, nous prendrons soins de ne pas confondre croissance à un stade BBCH donné et développement d'un stade BBCH à une autre à travers la saison, développement implicite à la formation des courbes de dilution critique de l'N selon Justes et al. 1994.

*Avantages apportés et activité inventive*

Établir une courbe Nc = $aPS^{-b}$ plutôt que Nc = $aPH^{-b}$ est en soi inédit. Cela dit, les valeurs empiriques de Nc sont maintenant établies non sur la base de courbes Nc : MS à la façon de Justes et al. 1994, approche maintenant classique, mais sur la base de seuils minimax asymptotiques des composantes P1 et des teneurs N1 et N2 d'une graminée spécifique quelconque. Les PS à ces valeurs de P1, N1 et N2 minimax et donc critiques en ce sens, sont par la suite rétro-calculés, chacune de ces séries étant établies à partir de fonctions logistiques asymptotiques particulières à chacune des séries de températures ambiantes de croissance particulières. La courbe Nc = $aPS^{-b}$ est ainsi constituée d'autant de points Nc : PS qu'il y a de telles fonctions asymptotiques particulières.

Concrètement, il s'agit donc d'une méthode pour le diagnostique de l'état azoto - nutritionnel de cultures agronomiques, fourragères, horticoles et maraîchères par comparaison de leurs teneurs en Na mesurées de leurs matières sèches des parties aériennes (MSPA) à des teneurs en N critiques, dites Nc, pour cette même MSPA et capables d'assurer un indice de nutrition azotée (NNI) = Na/Nc ≈ 1,00 puisqu'ici que Na ≈ Nc, caractérisée en ce que ;
➢ les MSPA sont celles de plants individuels de poids spécifiques (PS) (*eg*. g/plant) et non de biomasse par unité de surface, voire par hectare, la valeur de Nc étant ici fonction de ce PS des plant individuels,
et quelle comprend les étapes suivantes ;

i) prélèvement d'une série de plant individuels pour fin de détermination de leurs poids (sec) spécifiques, PS (*eg*. g/plant),
ii) détermination de la teneur en N de la MSPA de ce plant individuel de PS déterminé,
iii) comparaison de cette teneur en N à une teneur critique, Nc, exprimée sur la même base de PS provenant d'une courbe de dilution critique de l'azote expressément en fonction de PS de plants individuels,
iv) détermination d'un indice de l'état azoto-nutritionnel en tant que Na/Nc caractéristique du plant individuel et donc, de la culture de laquelle il provient.

Les plants individuels de PS déterminés appartiennent aux espèces de cultures céréalières, telles que le blé, l'orge, l'avoine et le seigle, ou encore aux espèces de grandes cultures telles que le colza, le tournesol, le maïs, grain et ensilage.

La courbe de dilution critique de l'azote Nc expressément fonction de PS de plants individuels est obtenue comme suit ;

i) construction de courbes de cinétiques de croissance et de développement de la MSPA en présence de différentes conditions thermo - climatiques ;
iii) normalisation thermique des susdites cinétiques de croissance sur la base du cumul d'unités thermique (ut) en différentes conditions thermo - climatiques produisant *in fine* une gamme de MSPA différentes ;
iv) extrapolation des valeurs N1 et 2, et P1 et 2 correspondants aux teneurs en N et aux proportions relatives MSPA des composantes dites végétatives (1) et structurelles (2), respectivement, de la plante et sa culture ;
v) rétro - calcul des PS à partir des MSPA et des ut correspondants à ces valeurs de N1 et 2 et P1 et 2 pour chacune des susdites conditions thermo - climatiques à différentes MSPA produites à un moment donné mais nécessitant plus ou moins, selon le cas, d'ut cumulées.

La construction des courbes de croissance de la MSPA, la normalisation thermique, l'extrapolation des valeurs N1 et 2 et P1 et 2, et le rétro - calcul des PS à partir des MSPA et des ut correspondant à ces valeurs de Nc sont eux effectués comme suit ;

**a)** établir une courbe décrivant la cinétique de croissance (d'accumulation) de la MSPA selon le cumul d'unité thermiques (ut) (Figure 1), une cinétique thermique en sorte, par opposition à une plus conventionnelle cinétique temporelle, par exemple selon Caloin et Yu 1986. Cette cinétique thermique comprend avantageusement que la phase strictement logarithmique de croissance ;

**b)** calculer le taux de croissance selon ut, r(ut) (Figure 2), entant que première dérivée de la susdite cinétique thermique. Cette équation de r(ut) permet de déterminer le coefficient k1 lorsque qu'elle est extrapolée à ut ≈ 0,00 (Tableau 1). En effet, si on admet qu'en tout début de croissance, M1, i.e. la masse de MSPA végétativement active, est a peu près égale à M = M1 + M2 puisque M2 (la masse de MSPA structurelle et végétativement inactive), k1 peut être estimé à partir de la pente initiale des courbes de croissance ; a terme, il y aura autant de valeurs de k1 que de courbes de croissance à températures ambiantes différentes ;

**c)** établir une courbe de dilution de l'azote (total) de la MSPA, dilution a priori au sens de Justes et al. 1994, selon n(ut) (Figure 3) ;

**d)** mettre en relation n(ut) et r(ut) (Figure 4) de manière à obtenir une équation linéaire de type n(ut) = a · r(ut) + b, sachant que de Caloin et Yu (1986) N1 = a × k1 + N2, et que N2 = b (Tableau 2) ;

**e)** établir une relation de P1, la proportion de M1 dans la MSPA, en fonction de la MSPA (Figure 5), sachant que P1(ut) = r(ut) / k1 (Tableau 3). Pour ce faire il s'agit simplement de rétro - calculer pour chaque valeur du cumul d'ut la MSPA correspondante, et mettre cette dernière en relation avec P1 (Figure 5). L'équation P1(MSPA) à la Figure 5 peut maintenant servir à déterminer P1 pour différentes quantités de MSPA données, par exemple ici 5, 10, 15, 20 et 25 t/ha (Tableau 3); P2 est elle comme de raison = 1 - P1 ;

**f)** connaissant ainsi N1 et N2, ainsi que P1 et P2, on calcul N, ou plus exactement ici Nc puisque l'indice de nutrition azoté (NNI ; Justes et al. 1994) a été maintenu à ≈1,00 +/- 0,10 tout au cours des susdites cinétique thermiques de croissance de la MSPA ;

$$Nc = [N1 \cdot P1] + [N2 \cdot P2]$$

Pour les susdites différentes MSPA il est ainsi possible de calculer autant de valeurs d'Nc (Tableau 4, Figure 7). Or, ces valeurs d'Nc correspondent elles à des ut qui peuvent être rétro - calculées à partir des équations à al Figure 3, et reconverties par les équations à la Figure 1 en MSPA effectivement produite pour ce cumul d'ut ;

**g**) enfin, on peut légitimement reconvertir ces MSPA (t/ha) en PS (g/plant) puisque nous connaissons la densité de peuplement (DENSITE ; cf. Stics, Brisson et al. 2008 ; Tableau 4) ; cette densité est dans le cas de tels modélisations homogènes, ce qui n'est pas nécessairement le cas avec lors de cinétiques in vivo / *in situ,* mais néanmoins variable selon la condition climato - thermique.

Les étapes **a)** à **g)** étant appliquées à chacune des dix (10) conditions climato - thermiques, et cela donc pour une gamme de MSPA (eg. 5, 10, 15, 20 et 25 t/ha), on peut ainsi produire une cinquantaine (50) de points - variables (Tableau 4) décrivant la nouvelle courbe Nc selon les poids spécifiques des plant individuels (g/plant) au sens de la présente invention (Figure 7), et cela par opposition au usuelles courbes Nc selon les poids de la MSPA (t/ha), par exemple selon Justes et al. 1994.

Notons que la fonction Nc selon PS est dégressive, Nc diminuant selon l'augmentation du PS du plant individuel, avantageusement selon une fonction puissance avec exposant négatif de type $Nc = aPS^{-b}$ lorsque a et b sont des coefficients empiriques spécifiques aux plants individuels de la culture en question en cours de diagnostique. De plus, la fonction Nc : PS est constituée de points variables provenant de cinétiques de croissances produisant *in fine* une gamme de MSPA superficielles et de teneurs en N de ces MSPA, avantageusement par hectare (*eg*. t-MSPA/ha), ces MSPA respectant lors de leurs croissance et développement un NNI = Na/Nc ≈ 1.00 +/- 0.10.

Ladite cinétique de croissance est transposée (transformée) en transformant l'axe temporelle, usuellement l'abscisse (*alias* axe des x) en axe *thermo - temporelle* comportant, part exemple, des unités thermiques (ut) au lieu des plus usuelles unités de temps (*eg.* jours, heures), les stades de développement des cultures produisant différentes MSPA *in fine* étant ainsi en principe les mêmes malgré (en dépit) de ces différentes productions de MSPA selon les différentes conditions climatiques de culture mais correspondant à un même cumul d'ut. On parle dans ce cas de NORMALISATION THERMIQUE : Les plants de PS donnés mais variables à un moment donné auront atteint à peu près les mêmes stades de développement phénologique BBCH. Les coefficients k1 (éq. 3, 4 et 6 de Caloin et Yu 1986) et b (éq. 9 et 10, op. *cit.*) selon la progression de ut, et non du temps journalier, respecteront ici cette condition puisque, presque par définition, la mesure d'ut, unités climatiques par excellence, est foncièrement liée à de tels développements phénologiques (*cf.* McMaster et Wilhelm 1997).

Les susdites cinétiques de croissance de la MSPA selon le cumul des unités thermiques respectant à tout moment NNI = Na/Nc ≈ 1,00 +/- 0,10 et permettant d'obtenir les coefficients k1 et b, ces derniers servent maintenant à calculer la progression selon le cumul d'ut de P1 = ut/kg et P2 = 1-P1, ainsi que N1 = a · k1 + N2, sachant que N2 = b et que k1 est le taux d'accumulation de la MSPA, r(ut), lorsque ut = 0, ou du moins au tout début de la phase accélérée de croissance de la MSPA. On parle dans ce cas EXTRAPOLATION CRITIQUE, k1 et b permettant de calculer N12 et P12, aux selon le cumul d'ut pour les différentes conditions climatiques. Les progressions de ces valeurs peuvent maintenant être rapporté par rapport à la MSPA superficielle, soit ici en t/ha, produites en présence de ces différentes conditions climatiques. Il est ainsi possible, pour une MSPA donnée, d'identifier les valeurs N12 et P12 correspondant pour les différentes conditions climatiques. Ces gammes de différentes valeurs de N12 et P12 selon les différentes conditions climatiques sont caractéristiques du potentiel de production de MSPA, et cela un peu comme le propose Justes et al. 1994 ; ces auteurs proposent plutôt le point d'inflexion dans la courbe relatant la progression de la MSPA par rapport à sa teneur en N, ce qui est foncièrement différent.

Enfin, pour chacune des conditions de culture ayant amenée *in fine* les différentes productions superficielles de MSPA, les cumuls d'ut correspondants sont ainsi rétro - calculés selon les susdites cinétiques thermiques, ces valeurs d'ut cumulés correspondant elles à des valeurs de MSPA données pouvant à leur tour être converties en autant de valeurs N et donc N1. En effet, notons que puisque Nc selon les susdites valeurs critiques va varier selon les conditions climatiques. Il est donc possible de rétro - calculer, et donc MSPA pour chacune de ces cinétiques thermiques, les Nc pour les conditions les plus fraîches étant en principe les plus faibles. Connaissant la densité de semis, il est ainsi possible de transformer MSPS en PS. Pour ce faire, il faut supposer que cette densité de semis est uniforme ; seule une modélisation dynamique de telles cinétiques, temporelles et/ou thermique, le permet, ou encore une a priori très homogène des graines ou plantules, en pots individuels et amovible par exemple (*cf*. Caloin et Yu 1986). Par exemple, ici les densités de peuplement (DENSITE ; Stics) varièrent de 250 en condition climatiques plutôt fraîches, à 190 en conditions plus chaudes (Figure 6).

## BRÈVE DESCRIPTION DES DESSINS ET FIGURES

**Figure 1** : Courbes des cinétiques climato - thermiques pour une culture de blé produisant *in fine* plus ou moins de matières sèches des parties aérienne (MSPA ; t/ha) selon une série de conditions climatiques plus ou moins chaudes, soit ici en moyenne annuel de 6 à 14 degrés C. Les progressions de MSPA sont rapportées par rapport au cumul d'unités thermique (ut ; en abscisse) dès le début de la phase logarithmique (Ln) de croissance selon les conditions climatiques. Les dix (10) équations MSPA = a · Ln(ut) - b décrivant ces progressions sont affichées de haut (6C) en bas (14C). A noter que, selon Caloin et Yu 1986, le taux de croissance, ici sur une base thermique cependant, est r = ut/a (*cf*. Figure 3), et que le coefficient k1 peut ainsi être défini à ut≈ 0, soit initialement par extrapolation des équations r(ut) ici à la Figure 3.

**Figure 2** : Courbes décrivant la diminution progressive du taux de croissance, r(ut), de la biomasse aérienne du blé tendre d'hiver en fonction des unités thermiques (ut) cumulées pour chacune des dix (10) conditions climat- - thermiques ; les équations sont de haut en bas celles pour les conditions les plus fraîches (6C) au plus chaudes (14C) ; les dix (10) coefficients k1 sont égalent r(ut) lorsque ut = 0 par extrapolation. Ces valeurs de r(ut) peuvent maintenant être mise en relation avec n(ut), i.e. %N de la MSPA (Figure 4).

**Figure 3** : Courbes décrivant la "dilution" des teneurs en N des MSPA selon le cumul des ut. Les ut étant foncièrement phénologiques, ces différentes teneurs en N à une valeur d'ut donnée correspondent à un même stade de développement, *eg*. BBCH ; ces courbes de dilution de l'N sont ainsi *normalisées.* Ces valeurs de N selon ut peuvent maintenant être mises en relation avec les valeurs de r(ut) (Figure 4). Du coup, il est possible de tirer de cette équation n(ut) = a · r(ut) + b, pour chacune des dix (10) conditions climato - thermiques, une valeur de N1 = a · k1 + b, sachant que N2 = b. Une fois P1 et P2 connus, nous pourront calculer pour chacune des conditions climat- thermiques N(Nc) = (N1 · P1) + (N2 · P2). A noter enfin, qu'encore une fois les dix (10) équations pour les conditions climato - thermiques les plus fraîches (6C) et de plus en chaudes sont affichées de haut en base sur le graphique.

**Figure 4 :** Courbes décrivant la relation entre n(ut) et r(ut) pour chacune des dix (10) conditions climato - thermiques ; les équations de haut en bas sont pour les conditions de plus en plus fraîches, soit en moyenne de 6 à 14C en moyenne annuelle. Les équations n(ut) = a · r(ut) + b permettent d'établir les coefficients a et b propres à chacune des dix (10) conditions thermiques, sachant que b = N2 et que a ·k1 + N2 = N1. Il est ainsi possible d'identifier une série de valeurs N1 et N2 particulière à des MSPA plus ou moins importantes selon les conditions climatiques. En combinaison avec les proportions P1 et P2 de la biomasse végétative (active) et structurelle, respectivement (Figure 5), on calcul Nc (puisque NNI est maintenue ≈ 1,00) pour les biomasses à un même stade de développement (BBCH) et cumul d'ut.

**Figure 5** : Diminution progressives des proportions actives (végétatives) de la MSPA, P1, par opposition à structurelles (P2 = 1 - P2) selon l'accumulation de la MSPA pour les dix (10) conditions climato - thermiques modélisées ; la progression de P2 est directement proportionnelle à la dégression de P1 et n'a pas à être rapportée graphiquement ici. À l'aide de ces dix (10) équations, une gamme de valeurs P1 et P2 à différentes valeurs de MSPA, *eg*. 5, 10, 15, 20 et 22,5 t/ha (Tableau 4, Figure 7) peuvent être calculées pour chacune des dix (10) conditions climatiques plus ou moins fraîches. Appliquées aux valeur N1 et N2 susmentionnées, et elles aussi calculées pour chacune des dix (10) conditions climato - thermiques, il est ainsi possible de calculer à partir de P1 et P2 une série de valeurs de N = Nc = (N1 · P1) + (N2 · P2) correspondant à chacune de ces *(eg.* cinq) MSPA. On obtient ainsi, ici une cinquantaine (5 × 10) de points variables Nc : MSPA convertissables par la suite en Nc : PS étant donnée l'homogénéité des densités de peuplement inhérentes à de telles modélisations (*cf.* Figure 6). A noter enfin qu'encore une fois les dix (10) équations des courbes en conditions climato - thermiques des plus fraîches (6C) au plus chaudes sont affichées de haut en bas.

**Figure 6 :** La densité de peuplement (DENSITE ; plants/$m^2$) selon la modélisation des cinétiques de croissance de la MSPA en différentes conditions climato - thermiques sont progressivement plus faibles, mais néanmoins homogènes. Il est donc exceptionnellement possible de convertir la MSPA en PS. Cette diminution de la densité de peuplement à températures ambiantes plus élevées est du au fait que les plants croissent plus rapidement, sont plus volumineux et occupent donc plus de place.

**Figure 7** : Courbes de dilution critique de l'azote des parties aériennes de plants individuels d'une culture de blé maintenus à un indice azoto - nutritionnel (NNI ; Justes et al. 1994) ≈ 1,00. Les plantules sont de poids spécifiques (PS ; g/plants), tandis la culture est de poids superficiel, ou hectoriel (PH ; t/ha). A noter que la courbe Nc : PS (losanges) est plus basse et moins abrupte que la courbe Nc : PH, ce qui est raisonnable. En effet, la dilution de l'azote des plants individuel ne dépendant que de leurs volumes internes et non de leurs stades phénologiques puisque les valeurs Nc ont été calculées à partir des coefficients a, b et k1 *normalisés thermiquement* selon les cinétiques thermiques (ut), les plants ayant cumulé un même nombre d'ut étant donc, par définition, au même stade phénologique (BBCH). Ces courbes de dilution Nc : PS peuvent maintenant être appliquées au diagnostique azoto - nutritionnel (via NNI) de plants individuels, sans avoir à extrapoler le poids par hectare de tels plants (Figure 8).

**Figure 8 :** Exemple d'application de l'invention avec deux séries de 26 plants individuels, "témoins" et "Traités", provenant de deux parcelles de blé tendre d'hiver (Alsace, mi-juin 2013 ; 1a nature du traitement est ici sans importance). Les indices de nutrition azotée (NNI ; Justes et al. 1994) pour le graphe du haut ont été calculé à partir des MSPA estimée par extrapolation d'un modèle logistique de la MSPA en fonction du poids spécifique (PS ; g/plant) moyen des plantules (*eg.* Deng et al. 2012, Yin et al. 2003, Weiner et Freckleton 2010, *etc.*) ; l'équation logistique en question est ici la suivante MSPA = 25 · [1 / (25 + 12,5 · exp(-0,25 · PS)] - 0,00. Pour le graphe du bas, les NNI, ou maintenant "pNNI" pour faire la distinction, sont calculés à partir des Nc pour les PS (g/plant) selon l'équation à la Figure 7. *In fine,* les corrélations entre l'azote du feuillage et les indices NNI selon les PS sont plus importantes que celles avec les NNI selon les PH ; cela est aussi vrai pour les autres teneurs élémentaires du feuillage (Figure 9).

**Figure 9 :** Les coefficients de détermination ($R^2$) entre les teneurs élémentaires du feuillage, y comprise celle de l'azote (Figure 8) et les indices pNNI selon les PS sont plus importantes que celles avec les indices NNI selon les PH (**t**-MSPA/ha). (Nb. Sont rapportés ici que les $R^2$ pour les modalités témoins (Tableau 5).

## MODE DE RÉALISATION PRÉFÉRÉ DE L'INVENTION

Il faut dans un premier temps générer une sérier de cinétiques de croissance de la matière sèche des parties aériennes (MSPA ; t/ha) de la culture pour la quelle on veut établir une courbe de dilution critique de l'azote (Nc ; % de la MSPA), ici et à titre d'exemple le blé tendre d'hiver (BTH), et cela en différentes conditions climatiques, avantageusement thermiques. Cette modulation thermique à l'avantage d'affecter plus uniformément tous les processus sol/plants qu'une modulation, par exemple, de l'incidence radieuse (mJ/$m^2$).

J'ai généré ce type de cinétiques thermique de la MSPA ici pour dix (10) températures ambiantes moyennes (annuelles) ; 6,0 6,6, 7,1, 8,0, 8,8, 9,5, 10,5, 12,0, 12,6 et 13,8 degrés C. Les températures moyennes minimax mensuelles sont ainsi ajustés, la pluviométrie (mm) et l'incidence radieuse étant elles inchangés. Pour générer ces cinétiques thermiques de la MSPA j'ai utilisé une modélisation des processus sol/plante informatisée dite Stics (Brisson et al. 2008 ; http://www6.paca.inra.fr/stics), et retenu à titre d'itinéraire technique le cas de figure (exemple) d'une culture de blé sur une campagne semée en octobre. Hormis les susdites modifications des fichiers climatologiques selon les dix (10) températures ambiantes, y compris minimax, les autres fichiers Stics nécessaires au pilotage des modélisations ont été utilisés tels que mis à disposition lors de l'installation du logiciel via http://www6.paca.inra.fr/stics.

Les susdites cinétiques de croissance de la MSPA en différentes conditions climato - thermiques peuvent être générées avec d'autres itinéraires, *in vivo* et/ou *in situ.* Il faut simplement s'assurer que l'accumulation de la MSPA (ut), et donc de n(ut), ne dépendent que de PS, et non de l'encombrement (ombrage) et autres phénomènes liés à la densité de population (peuplement).

En ce sens, l'effort de développement expérimental pourra maintenant porter sur l'élaboration de ce type de courbes, notamment à différentes températures ambiantes de croissances. On peut par exemple reproduire ces courbes pour diverses cultures via une série d'essais en cabinets de croissance à différentes températures, en s'assurant que la croissance soit ainsi découplée du stade de croissance. Concrètement, il faut donc éviter l'encombrement, et récolter les plants au même stade BBCH.

Encore une fois, la construction des courbes de croissance de la MSPA, la normalisation thermique, l'extrapolation des valeurs N1 et 2 et P1 et 2, et le rétro - calcul des PS à partir des MSPA et des ut correspondant à ces valeurs de Nc sont effectués comme suit, et cela donc, pour chacune de ces dix (10) conditions climato - thermiques ;

**a).** établir la cinétique de croissance de la MSPA selon le cumul d'unité thermiques (ut) (Figure 1), une cinétique thermique en sorte, par opposition à une plus conventionnelle cinétique temporelle, par exemple selon Caloin et Yu 1986, et avantageusement strictement logarithmique ;
**b).** calculer le taux de croissance selon ut, r(ut) (Figure 2), entant que première dérivée de la susdite cinétique cinétique thermique. Cette équation de r(ut) permet de déterminer le coefficient k1 lorsque qu'elle est extrapolée à ut ≈ 0,00 (Tableau 1) ;
**c).** établir une courbe de dilution de l'azote (total) de la MSPA, dilution a priori au sens de Justes et al. 1994, selon n(ut) (Figure 3) ;
**d).** mettre en relation n(ut) et r(ut) (Figure 4) de manière à obtenir une équation linéaire de type n(ut) = a · r(ut) + b, sachant que de Caloin et Yu (1986) N1 = a · k1 + N2, et que N2 = b (Tableau 2) ;
**e).** établir une relation de P1, la proportion de M1 dans la MSPA, en fonction de la MSPA (Figure 5), sachant que P1 (ut) = r(ut) / k1 (Tableau 3). Pour ce faire il s'agit simplement de rétro - calculer pour chaque valeur du cumul d'ut la MSPA correspondante, et mettre cette dernière en relation avec P1 (Figure 5). L'équation P1 (MSPA) à la Figure 5 peut maintenant servir à déterminer P1 pour différentes quantités de MSPA données, eg. de 5 à 25 t/ha (Tableau 3) ; P2 est elle comme de raison = 1 - P1 ;
**f).** connaissant N1 et N2, ainsi que P1 et P2, on calcul N, ou plus exactement ici Nc puisque l'indice de nutrition azoté (NNI ; Justes et al. 1994) a été maintenu à ≈1,00 +/- 0,10 tout au cours des susdites cinétique thermiques de croissance de la MSPA ;

$$Nc = [N1 \cdot P1] + [N2 \cdot P2]$$

(Pour ces différentes MSPA on calcul autant de valeurs d'Nc (Tableau 4, Figure 7), ces valeurs d'Nc correspondant à des ut qui peuvent être rétro - calculées à partir des équations à al Figure 3, et reconverties par les équations à la Figure 1 en MSPA effectivement produite pour ce cumul d'ut.)
**g).** enfin, on peut légitimement reconvertir ces MSPA (t/ha) en PS (g/plant) puisque nous connaissons la densité de peuplement (DENSITE ; Tableau 4), ici homogène, mais néanmoins variable selon la condition climato - thermique.

Les étapes a) à g) étant appliquées à chacune des dix (10) conditions climato - thermiques, et cela donc pour une gamme de MSPA (*eg*. 5, 10, 15, 20 et 25 t/ha), on produit ainsi une cinquantaine (50) de points - variables (Tableau 4) décrivant la nouvelle courbe Nc selon les poids spécifiques des plant individuels (g/plant) au sens de la présente invention (Figure 7), et cela par opposition au usuelles courbe Nc selon les poids de la MSPA (t/ha), par exemple selon Justes et al. 1994.

## APPLICATIONS BIOINDUSTRIELLES ET AGRONOMIQUES

Pour mener des essais comparatifs *in situ* sur une parcelle agricole, soit on détermine par estimation superficielle la biomasse par hectare (PH ; *eg*. t/ha), soit, au sens de la présente invention, on détermine le poids spécifique (PS ; g/plant) d'une série de plants individuels. Le PH permettra de déterminer l'indice de nutrition azotée (NNI) au sens habituel (Justes et al. 1994), et le PS de déterminer "pNNI" au sens de la présente invention.

**TABLEAU 1** : Valeurs des taux croissance r(ut), soit les premières dérivées des cinétiques de croissances selon le cumule des ut (Figure 1) de 800 à 1340 pour, soit essentiellement pendant la phase logarithmique de croissance pour les dix (10) conditions climatiques plus ou moins fraîches (i.e. de température moyennes de 6 à 14C. Ces différentes conditions climatiques vont ainsi générer une gamme de dix (10) coefficients k1 par extrapolation des équations décrivant ces dégressions de r(ut) (Figure 3) à ut = 0.

| UT | 6,0 ºC | 6,6 ºC | 7.1 ºC | 8.0 ºC | 8.8 ºC | 9.5 ºC | 10.5 ºC | 12.0 ºC | 12.6 ºC | 13.8 ºC |
|---|---|---|---|---|---|---|---|---|---|---|
| 800 | 0,0365 | 0,0368 | 0,0349 | 0,0338 | 0,0338 | 0,0318 | 0,0290 | 0,0249 | 0,0249 | 0,0241 |
| 815 | 0,0358 | 0,0361 | 0,0343 | 0,0332 | 0,0332 | 0,0312 | 0,0284 | 0,0244 | 0,0245 | 0,0237 |
| 830 | 0,0352 | 0,0355 | 0,0337 | 0,0326 | 0,0326 | 0,0307 | 0,0279 | 0,0240 | 0,0240 | 0,0232 |
| 845 | 0,0345 | 0,0349 | 0,0331 | 0,0320 | 0,0320 | 0,0301 | 0,0274 | 0,0236 | 0,0236 | 0,0228 |
| 860 | 0,0339 | 0,0343 | 0,0325 | 0,0315 | 0,0314 | 0,0296 | 0,0269 | 0,0232 | 0,0232 | 0,0224 |
| 875 | 0,0333 | 0,0337 | 0,0319 | 0,0309 | 0,0309 | 0,0291 | 0,0265 | 0,0228 | 0,0228 | 0,0221 |
| 890 | 0,0328 | 0,0331 | 0,0314 | 0,0304 | 0,0304 | 0,0286 | 0,0260 | 0,0224 | 0,0224 | 0,0217 |
| 905 | 0,0322 | 0,0325 | 0,0309 | 0,0299 | 0,0299 | 0,0281 | 0,0256 | 0,0220 | 0,0220 | 0,0213 |
| 920 | 0,0317 | 0,0320 | 0,0304 | 0,0294 | 0,0294 | 0,0277 | 0,0252 | 0,0217 | 0,0217 | 0,0210 |
| 935 | 0,0312 | 0,0315 | 0,0299 | 0,0289 | 0,0289 | 0,0272 | 0,0248 | 0,0213 | 0,0213 | 0,0206 |
| 950 | 0,0307 | 0,0310 | 0,0294 | 0,0285 | 0,0285 | 0,0268 | 0,0244 | 0,0210 | 0,0210 | 0,0203 |
| 965 | 0,0302 | 0,0305 | 0,0290 | 0,0280 | 0,0280 | 0,0264 | 0,0240 | 0,0206 | 0,0206 | 0,0200 |
| 980 | 0,0298 | 0,0301 | 0,0285 | 0,0276 | 0,0276 | 0,0260 | 0,0236 | 0,0203 | 0,0203 | 0,0197 |
| 995 | 0,0293 | 0,0296 | 0,0281 | 0,0272 | 0,0272 | 0,0256 | 0,0233 | 0,0200 | 0,0200 | 0,0194 |
| 1010 | 0,0289 | 0,0292 | 0,0277 | 0,0268 | 0,0268 | 0,0252 | 0,0229 | 0,0197 | 0,0197 | 0,0191 |
| 1025 | 0,0285 | 0,0287 | 0,0273 | 0,0264 | 0,0264 | 0,0248 | 0,0226 | 0,0194 | 0,0194 | 0,0188 |
| 1040 | 0,0281 | 0,0283 | 0,0269 | 0,0260 | 0,0260 | 0,0245 | 0,0223 | 0,0192 | 0,0192 | 0,0186 |
| 1055 | 0,0277 | 0,0279 | 0,0265 | 0,0257 | 0,0256 | 0,0241 | 0,0220 | 0,0189 | 0,0189 | 0,0183 |
| 1070 | 0,0273 | 0,0275 | 0,0261 | 0,0253 | 0,0253 | 0,0238 | 0,0216 | 0,0186 | 0,0186 | 0,0180 |
| 1085 | 0,0269 | 0,0271 | 0,0258 | 0,0249 | 0,0249 | 0,0235 | 0,0213 | 0,0184 | 0,0184 | 0,0178 |
| 1100 | 0,0265 | 0,0268 | 0,0254 | 0,0246 | 0,0246 | 0,0231 | 0,0211 | 0,0181 | 0,0181 | 0,0175 |
| 1115 | 0,0262 | 0,0264 | 0,0251 | 0,0243 | 0,0242 | 0,0228 | 0,0208 | 0,0179 | 0,0179 | 0,0173 |
| 1130 | 0,0258 | 0,0261 | 0,0247 | 0,0240 | 0,0239 | 0,0225 | 0,0205 | 0,0176 | 0,0176 | 0,0171 |
| 1145 | 0,0255 | 0,0257 | 0,0244 | 0,0236 | 0,0236 | 0,0222 | 0,0202 | 0,0174 | 0,0174 | 0,0169 |
| 1160 | 0,0252 | 0,0254 | 0,0241 | 0,0233 | 0,0233 | 0,0219 | 0,0200 | 0,0172 | 0,0172 | 0,0166 |
| 1175 | 0,0248 | 0,0251 | 0,0238 | 0,0230 | 0,0230 | 0,0217 | 0,0197 | 0,0170 | 0,0170 | 0,0164 |
| 1190 | 0,0245 | 0,0248 | 0,0235 | 0,0227 | 0,0227 | 0,0214 | 0,0195 | 0,0167 | 0,0167 | 0,0162 |
| 1205 | 0,0242 | 0,0244 | 0,0232 | 0,0225 | 0,0224 | 0,0211 | 0,0192 | 0,0165 | 0,0165 | 0,0160 |
| 1220 | 0,0239 | 0,0241 | 0,0229 | 0,0222 | 0,0222 | 0,0209 | 0,0190 | 0,0163 | 0,0163 | 0,0158 |
| 1235 | 0,0236 | 0,0239 | 0,0226 | 0,0219 | 0,0219 | 0,0206 | 0,0188 | 0,0161 | 0,0161 | 0,0156 |

(suite)

| UT | 6,0 ºC | 6,6 ºC | 7.1 ºC | 8.0 ºC | 8.8 ºC | 9.5 ºC | 10.5 ºC | 12.0 ºC | 12.6 ºC | 13.8 ºC |
|---|---|---|---|---|---|---|---|---|---|---|
| 1250 | 0,0233 | 0,0236 | 0,0224 | 0,0217 | 0,0216 | 0,0204 | 0,0185 | 0,0159 | 0,0159 | 0,0154 |
| 1265 | 0,0231 | 0,0233 | 0,0221 | 0,0214 | 0,0214 | 0,0201 | 0,0183 | 0,0157 | 0,0158 | 0,0153 |
| 1280 | 0,0228 | 0,0230 | 0,0218 | 0,0211 | 0,0211 | 0,0199 | 0,0181 | 0,0156 | 0,0156 | 0,0151 |
| 1295 | 0,0225 | 0,0227 | 0,0216 | 0,0209 | 0,0209 | 0,0196 | 0,0179 | 0,0154 | 0,0154 | 0,0149 |
| 1310 | 0,0223 | 0,0225 | 0,0213 | 0,0207 | 0,0206 | 0,0194 | 0,0177 | 0,0152 | 0,0152 | 0,0147 |
| 1325 | 0,0220 | 0,0222 | 0,0211 | 0,0204 | 0,0204 | 0,0192 | 0,0175 | 0,0150 | 0,0150 | 0,0146 |
| 1340 | 0,0218 | 0,0220 | 0,0209 | 0,0202 | 0,0202 | 0,0190 | 0,0173 | 0,0149 | 0,0149 | 0,0144 |
| k1 | 0,08461 | 0,08542 | 0,08102 | 0,07848 | 0,07838 | 0,07379 | 0,06717 | 0,05777 | 0,05778 | 0,05595 |

**TABLEAU 2** : Simulation de la dilution progressive des teneurs en N des MSPA (n(ut)) selon le cumul de 800 à 1340 unités thermiques (ut) (Figure 3). Ces valeurs de n (ut) peuvent maintenant être mise en relation avec celles de r(ut) au Tableau 1 (Figure 4). On obtient ainsi pour chacune des conditions climato - thermiques plus (6C) ou moins (14C) fraîches les coefficients a et b de l'équation n(ut) = a · r(ut) + b, sachant que (*cf*. Caloin et Yu 1986 b = N2 et N1 = a · k1 + N2.

| UT | 6,0ºC | 6,6 ºC | 7,1ºC | 8.0ºC | 8,8ºC | 9,5ºC | 10,5 ºC | 12,0 ºC | 12,6ºC | 13,8ºC |
|---|---|---|---|---|---|---|---|---|---|---|
| 800 | 0,0182 | 0,0175 | 0,0172 | 0,0187 | 0,0190 | 0,0190 | 0,0190 | 0,0178 | 0,0161 | 0,0190 |
| 815 | 0,0178 | 0,0173 | 0,0170 | 0,0184 | 0,0187 | 0,0187 | 0,0187 | 0,0176 | 0,0160 | 0,0187 |
| 830 | 0,0175 | 0,0170 | 0,0167 | 0,0181 | 0,0185 | 0,0184 | 0,0184 | 0,0174 | 0,0158 | 0,0184 |
| 845 | 0,0172 | 0,0168 | 0,0165 | 0,0179 | 0,0182 | 0,0181 | 0,0181 | 0,0172 | 0,0157 | 0,0181 |
| 860 | 0,0169 | 0.0165 | 0,0163 | 0,0176 | 0,0180 | 0.0178 | 0,0178 | 0,0170 | 0,0156 | 0.0178 |
| 875 | 0,0166 | 0,0163 | 0,0161 | 0,0174 | 0,0177 | 0,0176 | 0,0176 | 0,0168 | 0,0154 | 0,0176 |
| 890 | 0,0163 | 0,0161 | 0,0159 | 0,0171 | 0,0175 | 0,0173 | 0,0173 | 0,0167 | 0,0153 | 0,0173 |
| 905 | 0,0160 | 0,0158 | 0,0157 | 0,0169 | 0,0173 | 0,0171 | 0,0171 | 0,0165 | 0,0152 | 0,0171 |
| 920 | 0,0157 | 0,0156 | 0,0156 | 0,0167 | 0,0171 | 0,0168 | 0,0168 | 0,0163 | 0,0151 | 0,0168 |
| 935 | 0,0155 | 0,0154 | 0,0154 | 0,0164 | 0,0168 | 0.0166 | 0,0166 | 0,0162 | 0,0150 | 0,0166 |
| 950 | 0.0152 | 0.0152 | 0,0152 | 0,0162 | 0.0166 | 0,0163 | 0.0163 | 0,0160 | 0,0149 | 0.0163 |
| 965 | 0,0150 | 0,0150 | 0,0150 | 0,0160 | 0,0164 | 0,0161 | 0,0161 | 0,0159 | 0,0148 | 0,0161 |
| 980 | 0,0147 | 0,0148 | 0,0149 | 0,0158 | 0,0162 | 0,0159 | 0,0159 | 0,0157 | 0,0147 | 0,0159 |
| 995 | 0,0145 | 0,0146 | 0,0147 | 0,0156 | 0,0161 | 0,0157 | 0,0157 | 0,0156 | 0,0146 | 0,0157 |
| 1010 | 0,0143 | 0,0145 | 0,0146 | 0,0154 | 0,0159 | 0,0155 | 0,0155 | 0,0154 | 0,0145 | 0,0155 |
| 1025 | 0,0141 | 0,0143 | 0,0144 | 0,0153 | 0,0157 | 0,0153 | 0,0153 | 0,0153 | 0,0144 | 0,0153 |
| 1040 | 0,0138 | 0,0141 | 0,0143 | 0,0151 | 0,0155 | 0,0151 | 0,0151 | 0,0152 | 0,0143 | 0,0151 |
| 1055 | 0,0136 | 0,0139 | 0,0141 | 0,0149 | 0,0153 | 0,0149 | 0,0149 | 0,0150 | 0,0142 | 0,0149 |
| 1070 | 0,0134 | 0,0138 | 0,0140 | 0,0147 | 0,0152 | 0,0147 | 0,0147 | 0,0149 | 0,0141 | 0,0147 |
| 1085 | 0,0132 | 0,0136 | 0,0139 | 0,0146 | 0,0150 | 0,0145 | 0,0145 | 0,0148 | 0,0140 | 0,0145 |
| 1100 | 0,0131 | 0,0135 | 0,0137 | 0,0144 | 0,0149 | 0,0144 | 0,0144 | 0,0147 | 0,0139 | 0,0144 |
| 1115 | 0,0129 | 0,0133 | 0,0136 | 0,0143 | 0,0147 | 0,0142 | 0,0142 | 0,0145 | 0,0138 | 0,0142 |
| 1130 | 0,0127 | 0,0132 | 0,0135 | 0,0141 | 0,0145 | 0,0140 | 0,0140 | 0,0144 | 0,0137 | 0,0140 |
| 1145 | 0,0125 | 0,0130 | 0,0133 | 0,0140 | 0,0144 | 0,0139 | 0,0139 | 0,0143 | 0,0136 | 0,0139 |
| 1160 | 0,0124 | 0,0129 | 0,0132 | 0,0138 | 0,0143 | 0,0137 | 0,0137 | 0,0142 | 0,0136 | 0,0137 |
| 1175 | 0,0122 | 0,0128 | 0,0131 | 0,0137 | 0,0141 | 0,0135 | 0,0135 | 0,0141 | 0,0135 | 0,0135 |
| 1190 | 0,0120 | 0,0126 | 0,0130 | 0,0135 | 0,0140 | 0,0134 | 0,0134 | 0,0140 | 0,0134 | 0,0134 |
| 1205 | 0,0119 | 0,0125 | 0,0129 | 0,0134 | 0,0138 | 0,0132 | 0,0132 | 0,0139 | 0,0133 | 0,0132 |
| 1220 | 0,0117 | 0.0124 | 0,0127 | 0,0133 | 0,0137 | 0.0131 | 0.0131 | 0.0138 | 0,0132 | 0.0131 |
| 1235 | 0,0116 | 0,0122 | 0,0126 | 0,0131 | 0,0136 | 0,0130 | 0,0130 | 0,0137 | 0,0132 | 0,0130 |
| 1250 | 0,0114 | 0,0121 | 0,0125 | 0,0130 | 0,0135 | 0,0128 | 0,0128 | 0,0136 | 0,0131 | 0,0128 |

(suite)

| UT | 6,0ºC | 6,6 ºC | 7,1ºC | 8.0ºC | 8,8ºC | 9,5ºC | 10,5 ºC | 12,0 ºC | 12,6ºC | 13,8ºC |
|---|---|---|---|---|---|---|---|---|---|---|
| 1265 | 0,0113 | 0,0120 | 0,0124 | 0,0129 | 0,0133 | 0,0127 | 0,0127 | 0,0135 | 0,0130 | 0,0127 |
| 1280 | 0,0112 | 0,0119 | 0,0123 | 0,0127 | 0,0132 | 0,0126 | 0,0126 | 0,0134 | 0,0130 | 0,0126 |
| 1295 | 0,0110 | 0,0118 | 0,0122 | 0,0126 | 0,0131 | 0,0124 | 0,0124 | 0,0133 | 0,0129 | 0,0124 |
| 1310 | 0,0109 | 0.0117 | 0,0121 | 0,0125 | 0,0130 | 0.0123 | 0.0123 | 0.0132 | 0,0128 | 0.0123 |
| 1325 | 0,0108 | 0,0115 | 0,0120 | 0,0124 | 0,0129 | 0,0122 | 0,0122 | 0,0131 | 0,0128 | 0,0122 |
| 1340 | 0,0106 | 0,0114 | 0,0119 | 0,0123 | 0,0128 | 0,0121 | 0,0121 | 0,0130 | 0,0127 | 0,0121 |
| | | | | | | | | | | |
| a | 0,51267 | 0,41167 | 0,37442 | 0,46877 | 0,46024 | 0,54393 | 0,59754 | 0,47790 | 0.34130 | 0,71737 |
| N2 = b | 0,00054 | 0,00243 | 0,00418 | 0,00286 | 0,00352 | 0,00176 | 0,00176 | 0,00596 | 0,00768 | 0,00176 |
| N1 = ak1 + N2 | 0,04408 | 0,03759 | 0,03452 | 0,03965 | 0,03959 | 0,04190 | 0,04190 | 0,03357 | 0,02740 | 0,04190 |

**TABLEAU 3** : Valeurs des proportions P1 (et donc P2 = 1 - P1) de la MSPA comprenant de la biomasse végétative, ou active, par opposition à structurelle (*cf.* P2), ou inactive. La teneur en azote de P1 et N1, et celle de P2, N2. P1 va ainsi décroître à mesure que progresse le cumul d'ut, et donc la MSPA correspondante (Figure 5). Puisque P1 = r(ut)/k1 (*cf.* Caloin et Yu 1986), deux paramètres eux même fonctions que d'ut (*cf.* Tableau 1), P1 d'une MSPA donnée pour différentes conditions climato - thermiques, bien que variable, correspondra à des plants aux mêmes stades *phénologiques* BBCH. *Idem* pour P2. Ici, cinq (5) niveaux de MSPA sont proposés ; 5, 10, 15, 20 et 25 t/MSPA ; les valeurs de P1 en présence des différentes conditions climato - thermiques pour chacune de ces MSPA sont calculées à partir des équations à la Figure 5.

| UT | 6.0ºC | 6.6ºC | 7,1ºC | 8,0 ºC | 8.8ºC | 9,5 ºC | 10,5ºC | 12,0 ºC | 12.6ºC | 13,8 ºC |
|---|---|---|---|---|---|---|---|---|---|---|
| 800 | 0,431 | 0,431 | 0,431 | 0,431 | 0,431 | 0,431 | 0,431 | 0,431 | 0,431 | 0,431 |
| 815 | 0,423 | 0,423 | 0,423 | 0,423 | 0,423 | 0,423 | 0,423 | 0,423 | 0,423 | 0,423 |
| 830 | 0,415 | 0,415 | 0,416 | 0,415 | 0,415 | 0,415 | 0,415 | 0,415 | 0,416 | 0,415 |
| 845 | 0,408 | 0,408 | 0,408 | 0,408 | 0,408 | 0,408 | 0,408 | 0,408 | 0,408 | 0,408 |
| 860 | 0,401 | 0,401 | 0,401 | 0,401 | 0,401 | 0,401 | 0,401 | 0,401 | 0,401 | 0,401 |
| 875 | 0,394 | 0,394 | 0,394 | 0,394 | 0,394 | 0,394 | 0,394 | 0,394 | 0,394 | 0.394 |
| 890 | 0,387 | 0,387 | 0,367 | 0,387 | 0,387 | 0.387 | 0,387 | 0,387 | 0,388 | 0,387 |
| 905 | 0,381 | 0,381 | 0,381 | 0,381 | 0,381 | 0,381 | 0,381 | 0,381 | 0,381 | 0,381 |
| 920 | 0,375 | 0,375 | 0,375 | 0,375 | 0,375 | 0,375 | 0,375 | 0,375 | 0,375 | 0,375 |
| 935 | 0,369 | 0,369 | 0,369 | 0,369 | 0,369 | 0,369 | 0,369 | 0,369 | 0,369 | 0,369 |
| 950 | 0,363 | 0,363 | 0,363 | 0,363 | 0,363 | 0,363 | 0,363 | 0,363 | 0,363 | 0,363 |
| 965 | 0,357 | 0,357 | 0,357 | 0,357 | 0,357 | 0,357 | 0,357 | 0,357 | 0,357 | 0,357 |
| 980 | 0,352 | 0,352 | 0,352 | 0,352 | 0,352 | 0,352 | 0,352 | 0,352 | 0,352 | 0,352 |
| 995 | 0,347 | 0,347 | 0,347 | 0,347 | 0,347 | 0,347 | 0,347 | 0,347 | 0,347 | 0,347 |
| 1010 | 0,341 | 0,341 | 0,341 | 0,341 | 0,341 | 0,341 | 0,341 | 0,341 | 0,341 | 0,341 |
| 1025 | 0,336 | 0,336 | 0,336 | 0,336 | 0,336 | 0,336 | 0,336 | 0,336 | 0,336 | 0,336 |
| 1040 | 0,332 | 0.332 | 0,332 | 0,332 | 0,332 | 0.332 | 0.332 | 0,332 | 0,332 | 0.332 |
| 1055 | 0,327 | 0,327 | 0,327 | 0,327 | 0,327 | 0,327 | 0,327 | 0,327 | 0,327 | 0,327 |
| 1070 | 0,322 | 0,322 | 0,322 | 0,322 | 0,322 | 0,322 | 0,322 | 0,322 | 0,322 | 0,322 |
| 1085 | 0,318 | 0,318 | 0,318 | 0,318 | 0,318 | 0,318 | 0,318 | 0,318 | 0,318 | 0,318 |
| 1100 | 0,313 | 0,313 | 0,314 | 0,314 | 0,314 | 0,313 | 0,313 | 0,314 | 0,314 | 0,313 |
| 1115 | 0,309 | 0,309 | 0,309 | 0,309 | 0,309 | 0,309 | 0,309 | 0,309 | 0,309 | 0,309 |
| 1130 | 0,305 | 0,305 | 0,305 | 0,305 | 0,305 | 0,305 | 0,305 | 0,305 | 0,305 | 0,305 |
| 1145 | 0,301 | 0,301 | 0,301 | 0,301 | 0,301 | 0,301 | 0,301 | 0,301 | 0,301 | 0,301 |
| 1160 | 0,297 | 0,297 | 0,297 | 0,297 | 0,297 | 0,297 | 0,297 | 0,297 | 0,297 | 0,297 |
| 1175 | 0,293 | 0,293 | 0,294 | 0,293 | 0,293 | 0,293 | 0,293 | 0,293 | 0,294 | 0,293 |
| 1190 | 0,290 | 0,290 | 0,290 | 0,290 | 0,290 | 0,290 | 0,290 | 0,290 | 0,290 | 0,290 |
| 1205 | 0,286 | 0,286 | 0,286 | 0,286 | 0,286 | 0,286 | 0,286 | 0,286 | 0,286 | 0,286 |

(suite)

| UT | 6.0ºC | 6.6ºC | 7,1ºC | 8,0 ºC | 8.8ºC | 9,5 ºC | 10,5ºC | 12,0 ºC | 12.6ºC | 13,8 ºC |
|---|---|---|---|---|---|---|---|---|---|---|
| 1220 | 0,283 | 0,283 | 0,283 | 0,283 | 0,283 | 0,283 | 0,283 | 0,283 | 0,283 | 0,283 |
| 1235 | 0,279 | 0,279 | 0,279 | 0,279 | 0,279 | 0,279 | 0,279 | 0,279 | 0,279 | 0,279 |
| 1250 | 0,276 | 0,276 | 0,276 | 0,276 | 0,276 | 0,276 | 0,276 | 0,276 | 0,276 | 0,276 |
| 1265 | 0,273 | 0,273 | 0,273 | 0,273 | 0,273 | 0,273 | 0,273 | 0,273 | 0,273 | 0,273 |
| 1280 | 0,269 | 0,269 | 0,269 | 0,269 | 0,269 | 0,269 | 0,269 | 0,269 | 0,269 | 0,269 |
| 1295 | 0,266 | 0,266 | 0,266 | 0,266 | 0,266 | 0,266 | 0,266 | 0,266 | 0,266 | 0,266 |
| 1310 | 0,263 | 0,263 | 0,263 | 0,263 | 0,263 | 0,263 | 0,263 | 0,263 | 0,263 | 0,263 |
| 1325 | 0,260 | 0,260 | 0,260 | 0,260 | 0,260 | 0,260 | 0,260 | 0,260 | 0,260 | 0,260 |
| 1340 | 0,257 | 0,257 | 0,257 | 0,257 | 0,257 | 0,257 | 0,257 | 0,257 | 0,257 | 0,257 |
| P1 : 5 tMSPA | 0,56214 | 0,53758 | 0,53861 | 0,53937 | 0,52927 | 0,53244 | 0,54194 | 0,57323 | 0,57153 | 0,5423 |
| P1 : 10 tMSPA | 0,47914 | 0,45753 | 0,45426 | 0,45287 | 0,44387 | 0,44174 | 0,44134 | 0,45268 | 0,45133 | 0,4236 |
| P1:15 tMSPA | 0,40614 | 0,38698 | 0,38041 | 0,37787 | 0,36997 | 0,36404 | 0,35624 | 0,35313 | 0,35213 | 0,3274 |
| P1 : 20 tMSPA | 0,34314 | 0,32593 | 0,31706 | 0,31437 | 0,30757 | 0,29934 | 0,28664 | 0,27458 | 0,27393 | 0,2537 |
| P1 : 25 tMSPA | 0,29014 | 0,27438 | 0,26421 | 0,26237 | 0,25667 | 0,24764 | 0,23254 | 0,21703 | 0,21673 | 0,2025 |

**TABLEAU 4** : Résumé des modélisations Stics™ ayant servies à la génération des quelques 50 points variables Nc : PS selon la présente invention, par opposition aux Nc : PH selon Justes et al. 1994. A partir de la Figure 5, on observe (calcul) P1 lorsque 5, 10, 15, 20 et 25 t/ha de MSPA sont accumulées pour chacune dix (10) des conditions climato - thermiques plus ou moins fraîches. Ces valeurs de P1 (Tableau 3), et donc P2 = 1-P1, en combinaison avec les valeurs de N1 et N2 pré - calculées pour chacune desdites conditions climato - thermiques (Tableau 2) permettent le calcul d'un cinquantaine de valeurs Nc : PS ; voir les étapes (a) à (g) *supra*.

| T Celsius moyenne | MSAP : P1 | UT | MSPA | plants/m2 | PS | Nc : PS | Nc : PH |
|---|---|---|---|---|---|---|---|
| 6,0 | 5 | 590 | 4,32 | 250 | 1,73 | 0,0249 | 0,0280 |
| 6,6 | 5 | 632 | 4,88 | 245 | 1,99 | 0,0213 | 0,0265 |
| 7,1 | 5 | 623 | 4,55 | 235 | 1,94 | 0,0205 | 0,0274 |
| 8.0 | 5 | 628 | 4,84 | 220 | 2,20 | 0,0227 | 0,0266 |
| 8,8 | 5 | 639 | 4,75 | 205 | 2,32 | 0,0226 | 0,0269 |
| 9,5 | 5 | 641 | 5,02 | 200 | 2,51 | 0,0231 | 0,0262 |
| 10.5 | 5 | 558 | 2,26 | 195 | 1,16 | 0,0235 | 0,0373 |
| 12,0 | 5 | 572 | 4,34 | 193 | 2,25 | 0,0218 | 0,0280 |
| 12,6 | 5 | 562 | 3,91 | 191 | 2,05 | 0,0190 | 0,0293 |
| 13,8 | 5 | 475 | | 190 | | 0,0235 | |
| 6,0 | 10 | 686 | 8,70 | 250 | 3,48 | 0,0213 | 0,0206 |
| 6,6 | 10 | 749 | 9,92 | 245 | 4,05 | 0,0185 | 0,0194 |
| 7,1 | 10 | 752 | 9,81 | 235 | 4,18 | 0,0180 | 0,0195 |
| 8,0 | 10 | 757 | 9,88 | 220 | 4,49 | 0,0195 | 0,0194 |
| 8,8 | 10 | 772 | 9,87 | 205 | 4,81 | 0,0195 | 0,0194 |
| 9,5 | 10 | 778 | 9,95 | 200 | 4,98 | 0,0195 | 0,0194 |
| 10,5 | 10 | 738 | 8,72 | 195 | 4,47 | 0,0195 | 0,0205 |
| 12,0 | 10 | 752 | 9,78 | 193 | 5,07 | 0,0185 | 0,0195 |
| 12,6 | 10 | 750 | 9,68 | 191 | 5,07 | 0,0166 | 0,0196 |
| 13,8 | 10 | 705 | 7,23 | 190 | 3,80 | 0,0188 | 0,0223 |
| 6.0 | 15 | 801 | 13,21 | 250 | 5,29 | 0,0182 | 0.0171 |
| 6,6 | 15 | 891 | 15,03 | 245 | 6,14 | 0,0160 | 0,0161 |
| 7,1 | 15 | 907 | 15,07 | 235 | 6,41 | 0,0157 | 0,0161 |
| 8.0 | 15 | 913 | 14,97 | 220 | 6,80 | 0.0168 | 0,0162 |
| 8.8 | 15 | 933 | 15,00 | 205 | 7,32 | 0,0169 | 0,0162 |
| 9,5 | 15 | 948 | 14,97 | 200 | 7,49 | 0,0164 | 0,0162 |
| 10,5 | 15 | 982 | 15,33 | 195 | 7,86 | 0,0161 | 0,0160 |
| 12,0 | 15 | 981 | 15,08 | 193 | 7,81 | 0,0157 | 0,0161 |
| 12,6 | 15 | 985 | 15,10 | 191 | 7,90 | 0,0146 | 0,0161 |
| 13,8 | 15 | 1055 | 15,00 | 190 | 7,89 | 0,0149 | 0,0162 |
| 6,0 | 20 | 937 | 17,80 | 250 | 7,12 | 0,0154 | 0,0150 |
| 6,6 | 20 | 1060 | 20,14 | 245 | 8,22 | 0,0139 | 0,0142 |
| 7.1 | 20 | 1091 | 20,22 | 235 | 8,60 | 0,0138 | 0,0142 |
| 8,0 | 20 | 1099 | 19,98 | 220 | 9,08 | 0,0144 | 0,0142 |
| 8,8 | 20 | 1123 | 20,00 | 205 | 9,76 | 0,0146 | 0,0142 |
| 9.5 | 20 | 1153 | 19,95 | 200 | 9,97 | 0,0138 | 0,0143 |
| 10,5 | 20 | 1303 | 21,89 | 195 | 11,22 | 0,0133 | 0.0137 |
| 12,0 | 20 | 1253 | 19,95 | 193 | 10.34 | 0.0135 | 0,0142 |
| 12,6 | 20 | 1254 | 19,91 | 191 | 10,42 | 0,0131 | 0.0143 |
| 13,8 | 20 | 1553 | 22,46 | 190 | 11,82 | 0,0119 | 0,0135 |

(suite)

| T Celsius moyenne | MSAP : P1 | UT | MSPA | plants/m2 | PS | Nc : PS | Nc : PH |
|---|---|---|---|---|---|---|---|
| 6.0 | 25 | 1094 | 22,33 | 250 | 8,93 | 0,0131 | 0,0136 |
| 6.6 | 25 | 1255 | 25.12 | 245 | 10.25 | 0,0121 | 0,0129 |
| 7.1 | 25 | 1299 | 25,10 | 235 | 10.68 | 0,0122 | 0.0129 |
| 8.0 | 25 | 1310 | 24,73 | 220 | 11,24 | 0,0125 | 0,0130 |
| 8.8 | 25 | 1336 | 24,69 | 205 | 12,05 | 0,0128 | 0,0130 |
| 9.5 | 25 | 1387 | 24,65 | 200 | 12.32 | 0.0117 | 0,0130 |
| 10,5 | 25 | 1698 | 28.02 | 195 | 14.37 | 0,0111 | 0,0123 |
| 12,0 | 25 | 1538 | 24,05 | 193 | 12,46 | 0,0120 | 0,0131 |
| 12.6 | 25 | 1524 | 23.80 | 191 | 12.46 | 0.0120 | 0.0132 |
| 13.8 | 25 | 2161 | 28,83 | 190 | 15,18 | 0.0099 | 0,0121 |

On pourrait soit échantillonner la superficie et transformer cette biomasse par unité de surface, généralement de l'ordre de quelques mètres carrés, en tonnes / hectare (t/ha), soit transformer le PS des plants en t/ha à l'aide d'une équation logistique tenant compte des relations allométriques entre plants plus ou moins grands. Cela dit, l'estimation de PH nécessite bel et bien la transformation d'une mesure approximative par échantillonnage en PH, cette transformation étant source d'imprécisions et d'erreurs. Au contraire, la détermination de PS et pNNI ne nécessitent pas ces transformations puisque pNNI peut être estimé directement de PS selon la courbe Nc : PS à la Figure 7. Notons que cette nouvelle courbe Nc est elle aussi spécifiquement universelle.

Nous avons donc déterminé des corrélations entre les variables élémentaires N, P, K, Ca et Mg, fluométriques et phytopathométriques (iPahtos) par rapport à NNI et pNNI. Pour ce faire, nous avons procédé comme suit ;

➢ échantillonnage des plants individuels : De deux parcelles, témoin et traitée, de blé tendre d'hiver semés à l'automne 2012 nous avons nous avons prélevées les 14 juin et 3 juillet 2013 treize (13) plants aux stades, approximativement, BBCH 62-65, soit un total de 26 plants. L'état phytosanitaire de ces plants a été immédiatement évalué selon un certain indice phytosanitaire, iPahtos, sur une échelle de 0 (sans infestations) et 10 (complètement infesté) ; seules l'état de cinq (5) dernière feuilles (à partir de la canopée) ont été considérées. Dans la foulée, l'indice fluométrique Fv/Fm (i.e. rendement quantique maximal) à été déterminé, les plant séchés et analysé pour leurs teneurs en N, P, K, Ca, Mg et oligoéléments (B, Fe, Mn, Cu et Zn ; laboratoire Sadef, Alsace, France).

➢ calcul des PS et PH : Les poids spécifiques des matières sèches des partie aérienne (MSPA) de ces 2 x 2 x 13 = 52 étant exprimés en g/plant. La MSPA par hectare (PH ; t/ha) ont été estimées elles à l'aide d'une fonction logistique non linéaires asymptotique $PH = a \times [1 / (a + b \times \exp(-cPS))] - d$, dont les coefficients a, b, c et d sont, respectivement, les suivants ; 25, 12,5, 0,25 et 0,00. Dans les faits, cette transformation des PS en PH, et les relations allométriques qu'elles impliquent, fait l'objet de beaucoup de travaux publiés (eg. Deng et al. 2012 et 2012 bis et Watkinson 1984).

Nous espérions observer ainsi des corrélations plus serrées entre ces diverses variables et pNNI, ce qui semble être le cas (Tableau 5, Figures 8 et 9) ;

**TABLEAU 5** (Figure 9) : Coefficients de détermination (%$R^2$) des corrélations NNI ou pNNI et leurs variables correspondantes ; à titre de comparaison, la différence entre ces deux $R^2$ est indiquée.

| | Variable | %$R^2$ - NNI ; Variable | %$R^2$ - pNNI ; Variable | %$R^2$ - pNNI - NNI |
|---|---|---|---|---|
| témoin | FAN N | 43 | 67 | 24 |
| témoin | FAN P | 31 | 46 | 15 |
| témoin | FAN K | 25 | 48 | 23 |
| témoin | FAN Ca | 12 | 30 | 18 |
| témoin | FAN mg | 23 | 40 | 17 |
| témoin | Fv/Fm | 16 | 32 | 16 |
| témoin | iPathos | 12 | 25 | 13 |

(suite)

| | Variable | %$R^2$ - NNI ; Variable | %$R^2$ - pNNI ; Variable | %$R^2$ - pNNI - NNI |
|---|---|---|---|---|
| Traité | FAN N | 56 | 70 | 14 |
| Traité | FAN P | 59 | 60 | 1 |
| Traité | FAN K | 2 | 2,2 | 0 |
| Traité | FAN Ca | 1 | 2 | 1 |
| Traité | FAN mg | 36 | 39 | 3 |
| Traité | Fv/Fm | 10 | 14 | 4 |
| Traité | iPathos | 6 | 25 | 19 |

***Références, bibliographie et brevets pertinents***

BERMUDEZ ET MALLARINO 2004. CORN RESPONSE TO STARTER FERTILIZER AND TILLAGE ACROSS AND WITHIN FIELDS HAVING ,NO-TILL MANAGEMENT HISTORIES, AGRON, J. 96:776-785 (2004)

BERMUDEZ FT MALLARINO 2002. YIELD AND EARLY GROWTH RESPONSES TO STARTER FERTILIZER IN NO-TILL CORN ASSESSED WITH PRECISION AGRICULTURE TECHNOLOGIES. AGRON- J. 94:1024-1033 (2002)

CALOIN ET YU 1986. RELATION ENTRE DILUTION DE L'AZOTE ET DE CINÉTIQUE CROISSANCE CHEZ LES GRAMINÉES. AGRONOMIE 1986 6(2) : 167-174

CALOIN ET YU 1984. ANALYSIS OF THE TIME COURSE OF CHANGE IN NITROGEN CONTENT IN DACTYLIS GLOMERATA L. USING A MODEL OF PLANT GROWTH, ANNALS OF BOTANY 54, 69-76, 1984

DENG ET AL. 2012. MODELS AND TESTS OF OPTIMAL DENSITY AND MAXIMAL YIELD FOR CROP PLANTS. 15823-15828, doi: 10.1073/pnas.1210955109

DENG ET AL. 2012 BIS. INSIGHTS INTO PLANT SIZE-DENSITY RELATIONSHIPS FROM MODELS AND AGRICULTURAL CROPS, PAGES 8600-8605 | PNAS | MAY 29, 2012 | VOL. 109 | NO. 22.

JUSTES ET AL. 1994. DETERMINATION OF A CRITICAL NITROGEN DILUTION CURVE FOR WINTER WHEAT CROP. ANNALS OF BOTANY 74: 397-407

TREMBLAY, NICOLAS, ZHIJIE WANG & CARL BÉLEC (2009) PERFORMANCE OF DUALEX IN SPRING WHEAT FOR CROP NITROGEN STATUS ASSESSMENT, YIELD PREDICTION AND ESTIMATION OF SOIL NITRATE CONTENT, JOURNAL OF PLANT NUTRITION, 33:1, 57-70. DOI: 10.1080/01904160903391081

WATKINSON 1984, YIELD-DENSITY RELATIONSHIPS: THE INFLUENCE OF RESOURCE AVAILABILITY ON GROWTH AND SELF-THINNING IN POPULATIONS OF VULPIA FASCICULATA. ANNALS OF BOTANY 53, 469-482, 1984

Yao et al. 2014, DEVELOPMENT OF CRITICAL NITROGEN DILUTION CURVE IN RICE BASED ON LEAF DRY MATTER, EUROPEAN JOURNAL OF AGRONOMY, vol. 55, 22 janvier 2014 (2014-01-22), pages 20-28.

**Revendications**

**1.** Méthode pour le diagnostique de l'état azoto - nutritionnel de cultures agronomiques, fourragères, horticoles et maraîchères par comparaison des teneurs en azote, Na, des matières sèches des parties aériennes, MSPA, de plants individuels de poids sec spécifiques déterminés, PS, à des teneurs critiques en azote, Nc, pour cette même MSPA **caractérisée en ce que** ;
➢ les MSPA sont celles de plants individuels de PS ayant pour unité, par exemple, g/plant, et non de biomasse par unité de surface, voire par hectare, la valeur de Nc étant ici fonction de ce PS des plants individuels,
et quelle comprend les étapes suivantes ;

i) prélèvement d'une série de plants individuels pour fin de détermination de leurs PS,
ii) détermination de la teneur en azote de la MSPA de ce plant individuel de PS déterminé,
iii) comparaison de cette teneur en azote à une teneur critique, Nc, exprimée sur la même base de PS provenant d'une courbe de dilution critique de l'azote exprimée en fonction de PS de plants individuels, ladite fonction Nc selon PS étant elle constituée de points variables provenant de cinétiques de croissances produisant une gamme de MSPA superficielles et de teneurs en azote, avantageusement par hectare ayant ainsi, par exemple, l'unité t-MSPA/ha, ces MSPA respectant lors de leurs croissance et développement un indice azoto-nutritionnel, NNI, NNI = Na/Nc ≈ 1,00 +/-0,10.

iv) détermination d'un indice de l'état azoto - nutritionnel entant que Na/Nc caractéristique du plant individuel et donc, de la culture de laquelle il provient.

**2.** Méthode pour le diagnostique de l'état azoto - nutritionnel de cultures agronomiques, fourragères, horticoles et maraîchères selon la revendication précédente **caractérisée en ce que** les plants individuels de PS déterminés appartiennent aux espèces de cultures céréalières, telles que le blé, l'orge, l'avoine et le seigle.

**3.** Méthode pour le diagnostique de l'état azoto - nutritionnel de cultures agronomiques, fourragères, horticoles et maraîchères selon la revendication 1 **caractérisée en ce que** les plant individuels de PS déterminés appartiennent aux espèces de grandes cultures telles que le colza, le tournesol, le maïs, grain et ensilage.

**4.** Méthode pour le diagnostique de l'état azoto - nutritionnel de cultures agronomiques, fourragères, horticoles et maraîchères selon une quelconque des revendications précédentes **caractérisée en ce que** la courbe de dilution critique de l'azote Nc exprimée en fonction de PS de plants individuels est obtenue comme suit ;

i) construction de courbes de cinétiques de croissance et de développement de la MSPA en présence de différentes conditions thermo - climatiques ;
iii) normalisation thermique des susdites cinétiques de croissance sur la base du cumul d'unités thermiques, ut, en différentes conditions thermo - climatiques produisant *in fine* une gamme de MSPA différentes ;
iv) extrapolation des valeurs N1 et N2, et P1 et P2 correspondant aux teneurs en azote et aux proportions relatives MSPA des composantes dites végétatives et structurelles, respectivement, de la plante et sa culture ;
v) rétro - calcul des PS à partir des MSPA et des ut correspondant à ces valeurs de N1 et N2 et P1 et P2 pour chacune des susdites conditions thermo - climatiques à différentes MSPA produites à un moment donné mais nécessitant plus ou moins, selon le cas, d'ut cumulées.

**5.** Méthode pour le diagnostique de l'état azoto - nutritionnel de cultures agronomiques, fourragères, horticoles et maraîchères selon la revendication précédente **caractérisée en ce que** la construction des courbes de croissance de la MSPA, la normalisation thermique, l'extrapolation des valeurs N1 et N2 et P1 et P2, et le rétro - calcul des PS à partir des MSPA et des ut correspondant à ces valeurs de Nc sont effectués comme suit ;

a) établir une courbe décrivant la cinétique de croissance de la MSPA selon le cumul d'unités thermiques, ne décrivant avantageusement que la phase strictement logarithmique de croissance de la MSPA ;
b) calculer le taux de croissance selon ut, r(ut), en tant que première dérivée de la susdite cinétique cinétique thermique, cette équation de r(ut) permettant de déterminer un coefficient k1 lorsqu'elle est extrapolée à ut $\approx$ 0,00 ;
c) établir une courbe de dilution de l'azote total de la MSPA, dilution selon la teneur en azote d'un plant individuel, n(ut) ;
d) mettre en relation n(ut) et r(ut) de manière à obtenir une équation linéaire de type $n(ut) = a \cdot r(ut) + b$, sachant que $N1 = a \times k1 + N2$, et que $N2 = b$ ;
e) établir une relation de P1, la proportion de la masse de MSPA végétativement active, M1 dans la MSPA, en fonction de la MSPA, sachant que $P1(ut) = r(ut) / k1$ en rétro - calculant pour chaque valeur du cumul d'ut la MSPA correspondante, et mettre cette dernière en relation avec P1 de manière à déterminer ainsi P1 pour différentes quantités de MSPA données, par exemple ici 5, 10, 15, 20 et 25 t/ha, P2 étant elle comme de raison = 1 - P1 ;
f) connaissant ainsi N1 et N2, ainsi que P1 et P2, on calcule Nc puisque l'indice de nutrition azoté, NNI, a été maintenu à $\approx$1,00 +/- 0,10 tout au cours des susdites cinétiques thermiques de croissance de la MSPA ;

$$Nc = [N1 \cdot P1] + [N2 \cdot P2]$$

pour les susdites différentes MSPA, calculant ainsi autant de valeurs d'Nc, ces valeurs d'Nc correspondant elles à des ut qui peuvent être rétro - calculées et reconverties en MSPA effectivement produite pour ce cumul d'ut ;
g) enfin, connaissant la densité de peuplement, on reconvertit ces MSPA ayant pour unité, par exemple, t/ha, en PS ayant pour unité, par exemple, g/plant, cette densité étant dans le cas de telles modélisations homogènes, ce qui n'est pas nécessairement le cas lors de cinétiques in vivo / *in situ,* mais néanmoins variable selon la condition climato - thermique ;

les étapes a) à g) étant appliquées à chacune des dix conditions climato - thermiques, et cela donc pour une gamme de MSPA, on peut ainsi produire une multitude de points - variables décrivant la nouvelle courbe Nc selon les PS des plant individuels ayant pour unité, par exemple, g/plant, et cela par opposition aux courbes usuelles Nc selon les poids hectoriels, PH, de la MSPA avec pour unité t/ha.

6. Méthode pour le diagnostique de l'état azoto - nutritionnel de cultures agronomiques, fourragères, horticoles et maraîchères selon une quelconque des revendications précédentes **caractérisée en ce que** la fonction Nc selon PS est dégressive, Nc diminuant selon l'augmentation du PS du plant individuel, avantageusement selon une fonction puissance avec exposant négatif de type $Nc = aPS^{-b}$ lorsque a et b sont des coefficients empiriques spécifiques aux plants individuels de la culture en question en cours de diagnostique.

7. Méthode pour le diagnostique de l'état azoto - nutritionnel de cultures agronomiques, fourragères, horticoles et maraîchères selon une quelconque des revendications précédentes **caractérisée en ce que** ladite cinétique de croissance est transposée en transformant l'axe temporelle, usuellement l'abscisse en axe *thermo - temporelle* comportant, par exemple, des unités thermiques, ut, au lieu des plus usuelles unités de temps exprimées, par exemple, en jours ou heures, les stades de développement des cultures produisant différentes MSPA *in fine* étant ainsi en principe les mêmes malgré ces différentes productions de MSPA selon les différentes conditions climato - thermiques de culture mais correspondant à un même cumul d'ut.

8. Méthode pour le diagnostique de l'état azoto - nutritionnel de cultures agronomiques, fourragères, horticoles et maraîchères selon la revendication 5 **caractérisée en ce que** les susdites cinétiques de croissance de la MSPA selon le cumul des unités thermiques respectant à tout moment $NNI = Na/Nc \approx 1{,}00$ +/- 0,10 et permettant d'obtenir les coefficients k1 et b, ces derniers servent maintenant à calculer la progression selon le cumul d'ut de P1 = ut/kg et P2 = 1-P1, ainsi que $N1 = a \cdot k1 + N2$, sachant que N2 = b et que k1 est le taux d'accumulation de la MSPA, r(ut), lorsque ut = 0, ou du moins au tout début de la phase accélérée de croissance de la MSPA.

9. Méthode pour le diagnostique de l'état azoto - nutritionnel de cultures agronomiques, fourragères, horticoles et maraîchères selon la revendication 4 **caractérisée en ce que** pour chacune des conditions de culture ayant amenée *in fine* les différentes production superficielles de MSPA, les cumuls d'ut correspondants sont ainsi rétro - calculés selon les susdites cinétiques thermiques, ces valeurs d'ut cumulées correspondant elles à des valeurs de MSPA données pouvant à leur tour être converties en autant de valeurs correspondant aux teneurs en azote et donc N1.

## Patentansprüche

1. Methode zur Diagnose des Stickstoff-Nährstoff-Status von landwirtschaftlichen, Futter-, Gartenbau- und Gemüsekulturen durch Vergleich des Stickstoffgehalts, Na, der Trockenmassen der oberirdischen Teile, MSPA, von einzelnen Pflanzen mit bestimmten spezifischen Trockengewichten, PS, mit kritischen Stickstoffgehalten, Nc, für dieselben MSPA, **dadurch gekennzeichnet, dass**;
➢ die MSPA jene der einzelnen Pflanzen von PS sind, die als Einheit z. B. g/Pflanze und nicht 10 Biomasse pro Flächeneinheit oder sogar pro Hektar haben, wobei der Nc-Wert hier eine Funktion von diesem PS der einzelnen Pflanzen ist,
und welche die folgenden Schritte umfasst;

    i) Probenahme einer Reihe von Einzelpflanzen zur Bestimmung ihres PS,
    ii) Bestimmung des Stickstoffgehalts im MSPA dieser einzelnen Pflanzen mit ermitteltem PS,
    iii) Vergleich dieses Stickstoffgehalts mit einem kritischen Stickstoffgehalt, Nc, ausgedrückt auf der gleichen Basis von PS aus einer kritischen Stickstoff-Verdünnungskurve, ausgedrückt als Funktion des PS von einzelnen Pflanzen, wobei die zuvor genannte Nc-Funktion nach PS ihrerseits aus variablen Punkten besteht, die aus Wachstumskinetiken stammen, die einen Bereich von Oberflächen-MSPAs und Stickstoffgehalten erzeugen, vorteilhafterweise pro Hektar, so dass sie beispielsweise die Einheit t-MSPA/ha haben, wobei diese MSPAs während ihres Wachstums und ihrer Entwicklung einen Stickstoff-Nährstoff-Index, NNI, $NNI = Na/Nc \approx 1{,}00$ 0,10, einhalten,
    iv) Bestimmung eines Index für den Stickstoff-Nährstoff-Status als Na/Nc, der für die einzelne Pflanze und damit für die Kultur, aus der sie stammt, charakteristisch ist.

2. Methode zur Diagnose des Stickstoff-Nährstoff-Status von landwirtschaftlichen, Futter-, Gartenbau- und Gemüsekulturen gemäß dem vorhergehenden Anspruch, **dadurch gekennzeichnet, dass** die Einzelpflanzen mit ermitteltem PS zu den Arten der Getreidekulturen wie Weizen, Gerste und Hafer und Roggen gehören.

3. Methode zur Diagnose des Stickstoff-Nährstoff-Status von landwirtschaftlichen, Futter-, Gartenbau- und Gemüsekulturen gemäß Anspruch 1, **dadurch gekennzeichnet, dass** die Einzelpflanzen mit ermitteltem PS zu den Arten der Feldfrüchte wie Raps, Sonnenblume, Mais, Körner- und Silomais gehören.

4. Methode zur Diagnose des Stickstoff-Nährstoff-Status von landwirtschaftlichen, Futter-, Gartenbau- und Gemüsekulturen gemäß einem der vorhergehenden Ansprüche, **dadurch gekennzeichnet, dass** die kritische Verdünnungskurve von Stickstoff Nc, ausgedrückt als Funktion des PS einzelner Pflanzen, wie folgt erhalten wird:

i) Konstruktion von Kurven für die Wachstums- und Entwicklungskinetik der MSPA bei unterschiedlichen thermisch-klimatischen Bedingungen,
ii) thermische Normalisierung der oben genannten Wachstumskinetiken auf der Grundlage der kumulierten thermischen Einheiten, ut, bei verschiedenen thermo-klimatischen Bedingungen, was letztendlich eine Reihe von verschiedenen MSPAs ergibt,
iii) Extrapolation der Werte N1 und N2 sowie P1 und P2, die dem Stickstoffgehalt und den relativen MSPA-Anteilen der sogenannten vegetativen bzw. strukturellen Komponenten der Pflanze und ihrer Kultur entsprechen,
iv) Rückrechnung der PS aus den MSPAs und den ut, die diesen Werten von NI und N2 und PI und P2 entsprechen, für jede der oben genannten thermo-klimatischen Bedingungen für verschiedene MSPAs, die zu einem gegebenen Zeitpunkt produziert werden, aber je nach Fall mehr oder weniger kumulierte ut benötigen.

5. Methode zur Diagnose des Stickstoff-Nährstoff-Status von landwirtschaftlichen, Futter-, Gartenbau- und Gemüsekulturen gemäß dem vorhergehenden Ansprüche, **dadurch gekennzeichnet, dass** die Konstruktion der Wachstumskurven der MSPA, die thermische Normalisierung, die Extrapolation der Werte N1 und N2 sowie P1 und P2 und die Rückrechnung der PS ausgehend von den MSPA und den ut, die diesen Werten von entsprechen, wie folgt durchgeführt werden;

a) Erstellen einer Kurve, die die Wachstumskinetik der MSPA entsprechend der Kumulierung der thermischen Einheiten beschreibt, wobei vorteilhafterweise nur die streng logarithmische Phase des Wachstums der MSPA beschrieben wird;
b) Berechnung der Wachstumsrate gemäß ut, r(ut), als erste Ableitung der oben genannten thermischen Kinetik, wobei diese Gleichung von r(ut) die Bestimmung eines Koeffizienten kl ermöglicht, wenn sie auf ut bei 0,00 extrapoliert wird,
c) Erstellen einer Verdünnungskurve für den Gesamtstickstoff der MSPA, Verdünnung gemäß dem Stickstoffgehalt einer einzelnen Pflanze, n(ut),
d) Die Werte n(ut) und r(ut) in Beziehung setzen, um eine lineare Gleichung vom Typ n(ut) = a * r(ut) + b zu erhalten, wobei N1 = a x k1 + N2 und N2 = b ist;
e) Erstellen einer Beziehung von P1, dem Anteil der vegetativ aktiven Masse der MSPA, M1, in der MSPA, in Funktion der MSPA, wissend dass P1 (ut) = r(ut) / kl durch Rückrechnung für jeden Wert der ut-Kumulation der entsprechenden MSPA, und das in Beziehung setzen dieser letzteren mit P1, um so P1 für verschiedene gegebene Mengen MSPA zu bestimmen, z.B. hier 5, 10, 15, 20 und 25 t/ha, wobei P2 wie selbstverständlich = 1 - P1 ist;
f) dadurch sind N1 und N2 sowie P1 und P2 bekannt, woraus Nc berechnet wird, da der Stickstoffernährungsindex, NNI, während der gesamten oben genannten thermischen Wachstumskinetik der MSPA bei 1,00 +/- 0,10 gehalten wurde,

$$Nc = [N1 \cdot P1] + [N2 \cdot P2]$$

g) für die zuvor genannten verschiedenen MSPa, somit wenden ebensoviele Nc-Werte berechnet, diese Werte 5 des Nc wiederum entsprechen den ut, die zurückgerechnet und in die tatsächlich produzierte MSPA für diese kumulierte ut zurückverwandelt werden können,
h) schließlich werden in Kenntnis der Bestandsdichte diese MSPA z.B. mit der Einheit t/ha wieder in PS mit z.B. der Einheit g/Pflanze umgerechnet, wobei diese Dichte im Fall solcher Modellierungen homogen ist, was bei in vivo/in situ-Kinetiken nicht unbedingt der Fall ist, aber dennoch je nach klimatisch-thermischer Bedingung variabel ist,

wobei die Schritte a) bis g) für jede der zehn klimatisch-thermischen Bedingungen und somit für eine Reihe von MSPAs angewendet werden, wodurch eine Vielzahl von variablen Punkten erzeugt werden kann, die die neue Nc-Kurve gemäß den PS der einzelnen Pflanzen beschreibt und als Einheit z.B. g/Pflanze hat, im Gegensatz zu den üblichen Nc-Kurven gemäß den Hektargewichten, PH, der MSPA mit der Einheit t/ha.

**6.** Methode zur Diagnose des Stickstoff-Nährstoff-Status von landwirtschaftlichen, Futter-, Gartenbau- und Gemüsekulturen gemäß einem der vorhergehenden Ansprüche, **dadurch gekennzeichnet, dass** die Funktion Nc nach PS degressiv ist, wobei Nc mit zunehmendem PS der einzelnen Pflanze abnimmt, vorteilhafterweise nach einer Potenzfunktion mit negativem Exponenten vom Typ Nc = a · PS-b, wenn a und b für Einzelpflanzen spezifische empirische Koeffizienten sind, aus der jeweiligen Kultur im Lauf der Diagnostik.

**7.** Methode zur Diagnose des Stickstoff-Nährstoff-Status von landwirtschaftlichen, Futter-, Gartenbau- und Gemüsekulturen gemäß einem der vorhergehenden Ansprüche, **dadurch gekennzeichnet, dass** die genannte Wachstumskinetik umgestellt wird, durch Transformation der Zeitachse, üblicherweise der Abszisse, in die eine thermisch-temporäre Achse, die z.B. thermische Einheiten, ut, umfasst, anstatt der üblicher angegebenen Zeiteinheiten wie z.b. Tage oder Stunden, die Entwicklungsstadien der Kulturen produzieren andere MSPA, letztendlich sind sie somit im Prinzip die gleichen, trotz dieser unterschiedlichen MSPA-Produktion entsprechend den unterschiedlichen klimatisch-thermischen Bedingungen der Kultur, aber entsprechend einer gleichen Kumulierung von ut.

**8.** Methode zur Diagnose des Stickstoff-Nährstoff-Status von landwirtschaftlichen, Futter-, Gartenbau- und Gemüsekulturen gemäß dem Anspruch 5, **dadurch gekennzeichnet, dass** die oben genannten Wachstumskinetiken des MSPA gemäß der Kumulierung der thermischen Einheiten, die zu jedem Zeitpunkt NNI = Na/Nc ≈ 1.00 +/- 0,10 einhalten und zur Gewinnung der Koeffizienten kl und b dienen, diese letzteren nun zur Berechnung des Fortschritts gemäß der Kumulierung von ut von P1 = ut/kg und P2 = 1-P1 sowie N1 = a · k1 + N2, wissend, dass N2 = b und k1 die Akkumulationsrate von MSPA ist, wenn ut = 0 ist, oder zumindest ganz am Beginn der beschleunigten Wachstumsphase von MSPA steht.

**9.** Methode zur Diagnose des Stickstoff-Nährstoff-Status von landwirtschaftlichen, Futter-, Gartenbau- und Gemüsekulturen gemäß dem Anspruch 4, **dadurch gekennzeichnet, dass** für jede der Kulturbedingungen, die letztendlich zu den unterschiedlichen Oberflächenproduktionen von MSPA geführt haben, die Kumulationen der entsprechenden ut gemäß den oben genannten thermischen Kinetiken zurückgerechnet werden, wobei diese kumulierten ut-Werte ihrerseits gegebenen MSPA-Werten entsprechen, die ihrerseits in ebenso viele Werte umgewandelt werden können, die dem Stickstoffgehalt und somit N1 entsprechen.

## Claims

**1.** Method for diagnosing the azoto-nutritional status of agronomic, fodder, horticultural and vegetable crops by comparison of nitrogen, Na, dry matter contents of aerial parts, MSPA, of specific dry weight individual plants, PS, at critical nitrogen levels, Nc, for the same MSPA **characterized in that**;

➢ the MSPA are those of individual PS plants having as a unit, for example, g/plant, and not 10 of biomass per unit area, or even per hectare, the Nc value being here a function of this PS of the individual plants,

and which includes the following steps;

i) sampling of a series of individual seedlings for the purpose of determining their HCPs,
ii) determination of the nitrogen content of the MSPA of that individual plant of given PS,
iii) comparison of that nitrogen content to a critical, NC, content, expressed on the same basis of PS from a critical nitrogen dilution curve expressed as a function of PS of individual plants, that PS NC function being made up of variable points from growth kinetics producing a range of surface MSPA and nitrogen contents, advantageously per hectare thus having, for example, the unit t-MSPA/ha, these MSPA having during their growth and development an azoto-nutritional index, NNI, NNI = Na / Nc ≈ 1.00 +/- 0.10,
iv) determination of an index of the azoto-nutritional status as Na/Nc characteristic of the individual plant and therefore, from the culture from which it comes.

**2.** Method for diagnosing the azoto-nutritional status of agronomic, fodder, horticultural and vegetable crops according to the preceding claim **characterized in that** the individual plants of PS determined belong to cereal crop species, such as wheat, barley and oats and rye.

**3.** Method for diagnosing the azoto-nutritional status of agronomic, fodder, horticultural and vegetable crops according to claim 1 **characterized in that** the individual plants of PS determined belong to field crop species such as rapeseed, sunflower, maize, grain and silage.

4. Method for the diagnosis of the azoto-nutritional state of agronomic, fodder, horticultural and vegetable crops according to any of the preceding claims **characterized in that** the critical dilution curve of Nc nitrogen expressed as a function of PS of individual plants is obtained as follows,

   i) construction of growth and development kinetics curves of the MSPA in different thermo-climatic conditions,
   (ii) thermal normalisation of the above-mentioned growth kinetics on the basis of the accumulation of thermal units, ut, under different thermo-climatic conditions ultimately producing a range of different MSPA,
   (iii) extrapolation of the NI and N2 values, and PI and P2 corresponding to the nitrogen contents and the mspa relative proportions of the so-called vegetative and structural components, respectively, of the plant and its crop,
   (iv) retro - calculation of the PS from the MSPA and the ut corresponding to these values of NI and N2 and PI and P2 for each of the aforementioned thermo-climatic conditions at different MSPA at a given time but requiring more or less, as the case may be, cumulative ut.

5. Method for the diagnosis of the azoto-nutritional state of agronomic, fodder, horticultural and vegetable crops according to the preceding claim **characterized in that** the construction of the growth curves of the MSPA, the thermal normalization, the extrapolation of the N1 and N2 and P1 and P2 values, and the retro-calculation of PS from MSPA and the ut corresponding to these Nc values are carried out as follows ;

   a) establish a curve describing the growth kinetics of the MSPA according to the accumulation of thermal units, advantageously describing only the strictly logarithmic phase of growth of the MSPA,
   b) calculate the growth rate according to ut, r(ut), as the first derivative of the aforementioned thermal kinetics, this equation of r(ut) being used to determine a coefficient kl when extrapolated to ut to 0,00,
   c) establish a dilution curve of the total nitrogen of the MSPA, dilution according to the nitrogen content of an individual plant, n(ut),
   d) relate n(ut) and r(ut) so as to obtain a linear equation of type n(ut) = a • r(ut) + b, knowing that N1 = a x k1 + N2, and that N2 = b,
   e) establish a relationship of P1, the proportion of the mass of vegetatively active MSPA, M1 in the MSPA, as a function of the MSPA, knowing that P1 (ut) = r(ut) / kl in retro - calculating for each value of the accumulation of ut the corresponding MSPA, and put the latter in relation to P1 so as to determine P1 for different quantities of given MSPA, for example here 5, 10, 15, 20 and 25 t / ha, P2 being it as of reason = 1 - P1,
   f) thus knowing N1 and N2, as well as PI and P2, nc is calculated since the nitrogen nutrition index, NNI, was maintained at 1.00 +/- 0.10 throughout the aforementioned thermal kinetics of growth of the MSPA,

$$Nc = [N1 \cdot P1] + [N2 \cdot P2]$$

   for the aforementioned different MSPA, thus calculating as many values of Nc, these values 5 of Nc corresponding to ut which can be retro - calculated and reconverted into MSPA actually produced for this accumulation of ut,
   g) finally, knowing the population density, we convert these MSPA having for unit, for example, t/ha, in PS having for unit, for example, g/plant, this density being in the case of such homogeneous models, which is not necessarily the case during kinetics in vivo / in situ, but nevertheless variable according to the thermo-climatic condition,

   Steps a) to g) being applied to each of the ten thermo-climatic conditions for a range of MSPA, one can thus produce a multitude of points-variables describing the new Nc curve according to the PS of the individual plants having as unit, for example, g/plant, and this as opposed to the usual Nc curves according to the per hectare weights, PH, of the MSPA with for unit t/ha.

6. Method for the diagnosis of the azoto-nutritional state of agronomic, fodder, horticultural and vegetable crops according to any of the preceding claims **characterized in that** the Nc function according to PS is degressive, Nc decreasing according to the increase in the PS of the individual plant preferably according to a negative power function such as $Nc = a \cdot PS^{-b}$ where a and b are empirical coefficients specific to the individual plants of the crop in question being diagnosed.

7. Method for the diagnosis of the azoto-nutritional state of agronomic, fodder, horticultural and vegetable crops according to any of the preceding claims **characterized in that** said growth kinetics is transposed by transforming the time axis, usually the abscissa, into a thermo-temporal axis comprising, for example, thermal units, ut, instead of the usual units of time expressed, for example, in days or hours, the stages of development of crops producing different MSPA but ultimately the same despite these different MSPA as a function of the different thermo-climatic conditions of cultivation but corresponding to the same accumulation of ut.

8. Method for the diagnosis of the azoto-nutritional state of agronomic, fodder, horticultural and vegetable crops according to claim 5 **characterized in that** the aforementioned growth kinetics of the MSPA according to the accumulation of thermal units respecting at any time $NNI = Na/Nc \approx 1.00 +/- 0.10$ and yielding the k1 and b coefficients, the latter are now used to calculate the progression according to the accumulation of ut of $P1 = ut/kg$ and $P2 = 1-P1$, as well as $N1 = a \cdot k1 + N2$, knowing that $N2 = b$ and k1 is the rate of accumulation of the MSPA, when $ut = 0$, or at least at the very beginning of the accelerated phase of growth of the MSPA.

9. Method for the diagnosis of the azoto-nutritional state of agronomic, fodder, horticultural and vegetable crops according to claim 4 **characterized in that** for each of the growing conditions having ultimately led to the different aerial production of MSPA, the corresponding accumulations of ut are thus retro-calculated according to the aforementioned thermal kinetics, these cumulative ut values corresponding to given MSPA values that can in turn be converted into just as many N1 nitrogen contents estimates.

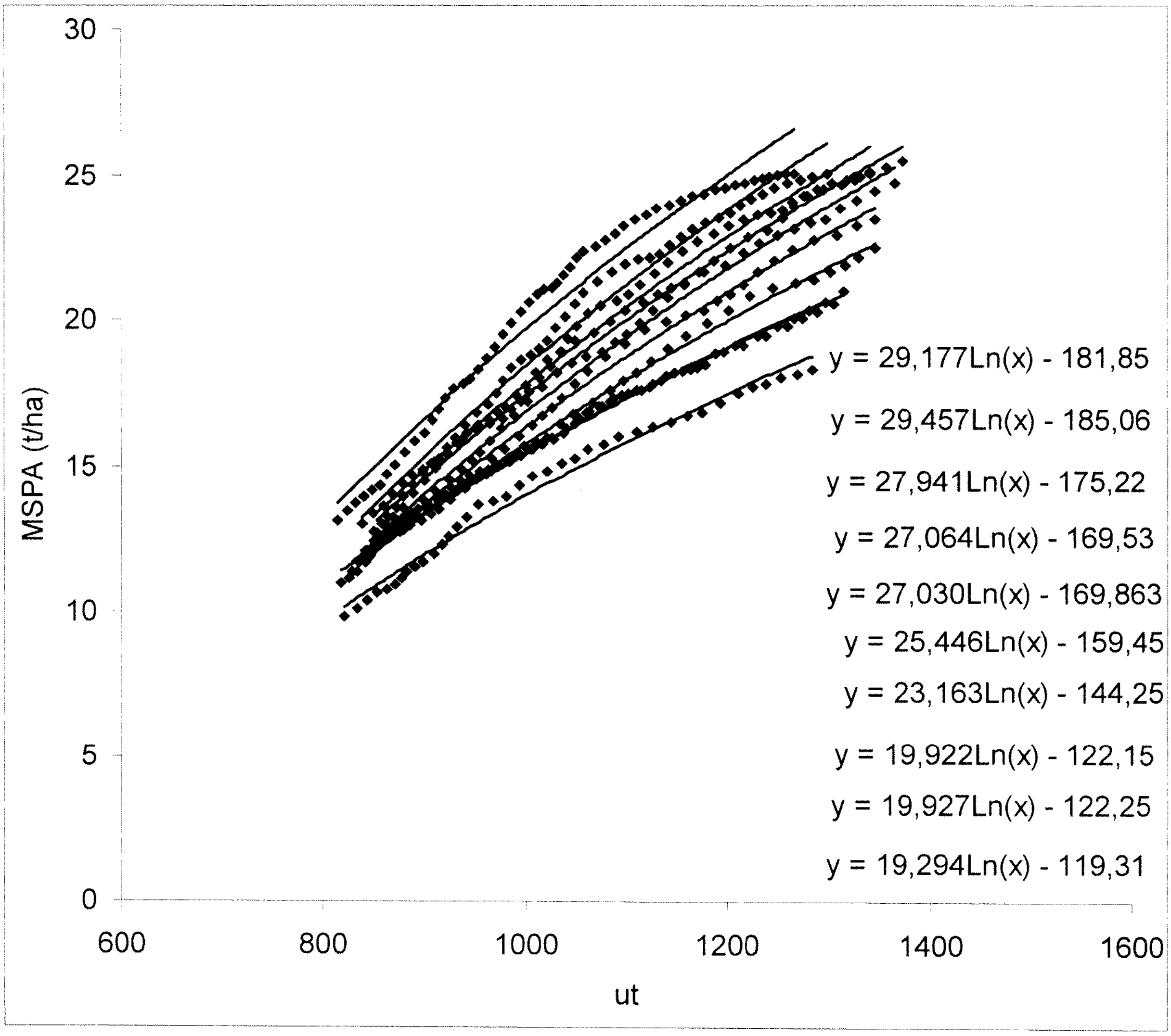
30
25
20
15
10
5
0
MSPA (t/ha)
600
800
1000
1200
1400
1600
ut
y = 29,177Ln(x) - 181,85
y = 29,457Ln(x) - 185,06
y = 27,941Ln(x) - 175,22
y = 27,064Ln(x) - 169,53
y = 27,030Ln(x) - 169,863
y = 25,446Ln(x) - 159,45
y = 23,163Ln(x) - 144,25
y = 19,922Ln(x) - 122,15
y = 19,927Ln(x) - 122,25
y = 19,294Ln(x) - 119,31

FIGURE 1 :

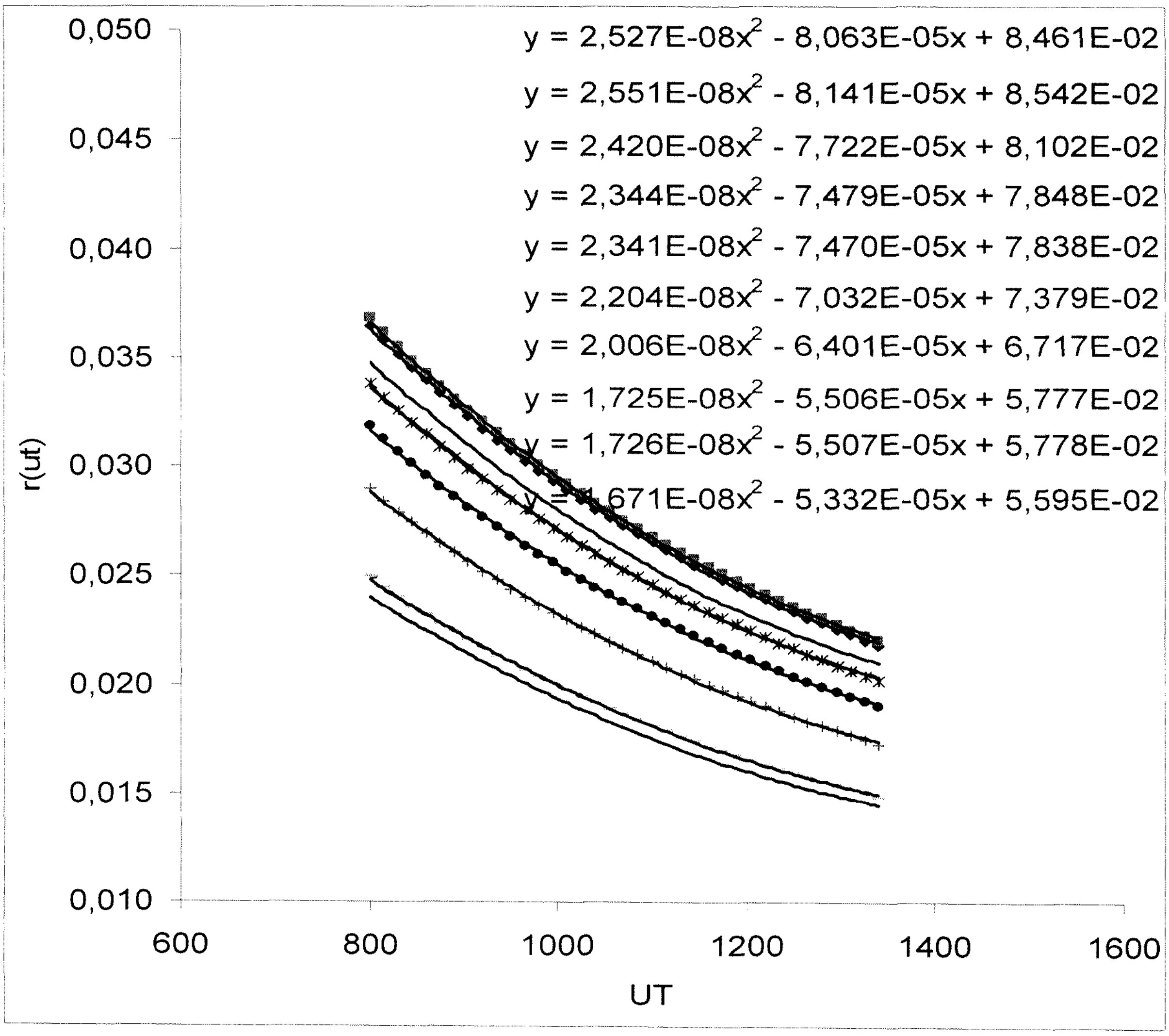
y = 2,527E-08x2 - 8,063E-05x + 8,461E-02
y = 2,551E-08x2 - 8,141E-05x + 8,542E-02
y = 2,420E-08x2 - 7,722E-05x + 8,102E-02
y = 2,344E-08x2 - 7,479E-05x + 7,848E-02
y = 2,341E-08x2 - 7,470E-05x + 7,838E-02
y = 2,204E-08x2 - 7,032E-05x + 7,379E-02
y = 2,006E-08x2 - 6,401E-05x + 6,717E-02
y = 1,725E-08x2 - 5,506E-05x + 5,777E-02
y = 1,726E-08x2 - 5,507E-05x + 5,778E-02
y = 1,671E-08x2 - 5,332E-05x + 5,595E-02
r(ut)
0,050
0,045
0,040
0,035
0,030
0,025
0,020
0,015
0,010
600
800
1000
1200
1400
1600
UT

FIGURE 2 :

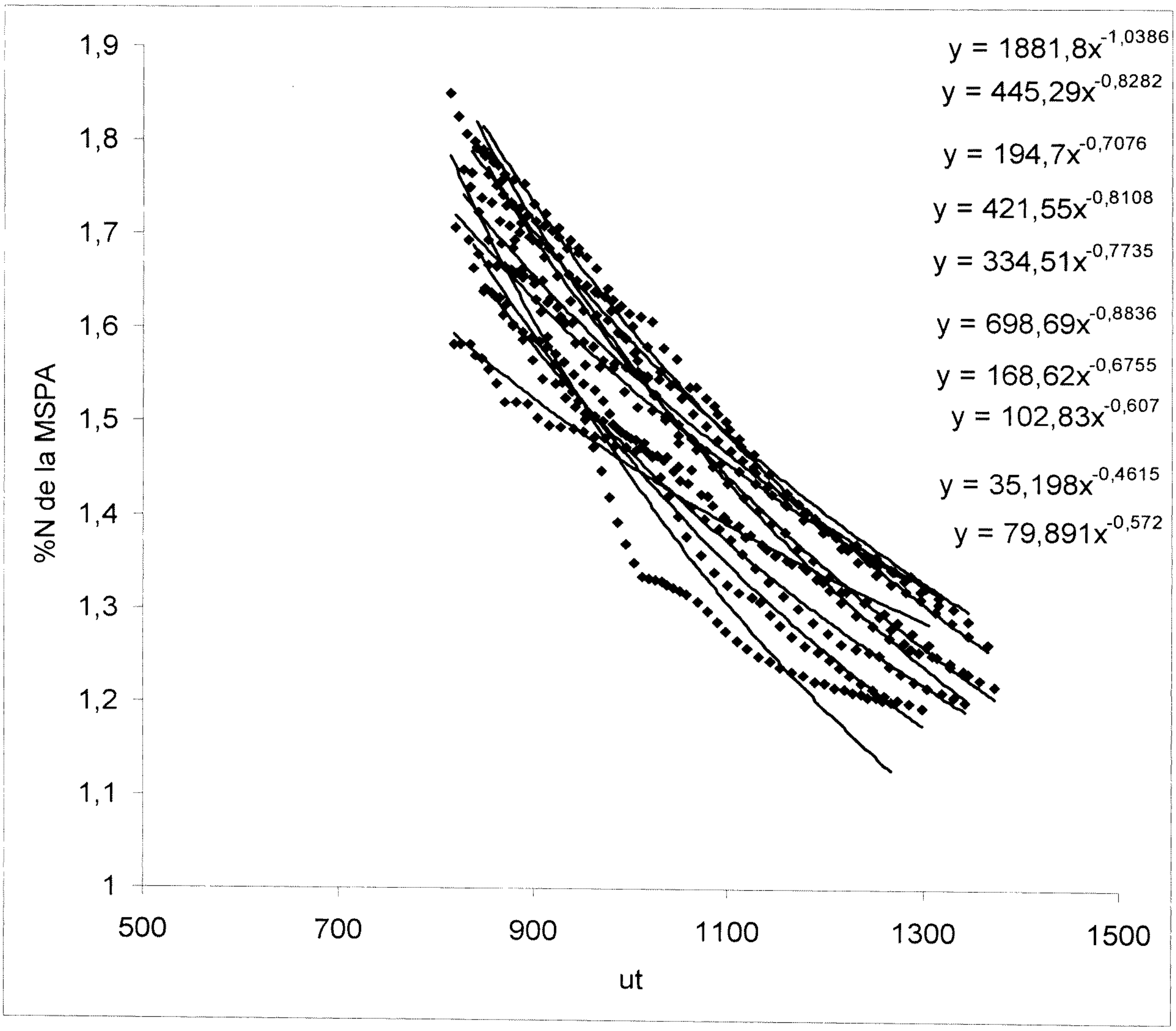
y = 1881,8x-1,0386
y = 445,29x-0,8282
y = 194,7x-0,7076
y = 421,55x-0,8108
y = 334,51x-0,7735
y = 698,69x-0,8836
y = 168,62x-0,6755
y = 102,83x-0,607
y = 35,198x-0,4615
y = 79,891x-0,572
%N de la MSPA
1,9
1,8
1,7
1,6
1,5
1,4
1,3
1,2
1,1
1
500
700
900
1100
1300
1500
ut

FIGURE 3 :

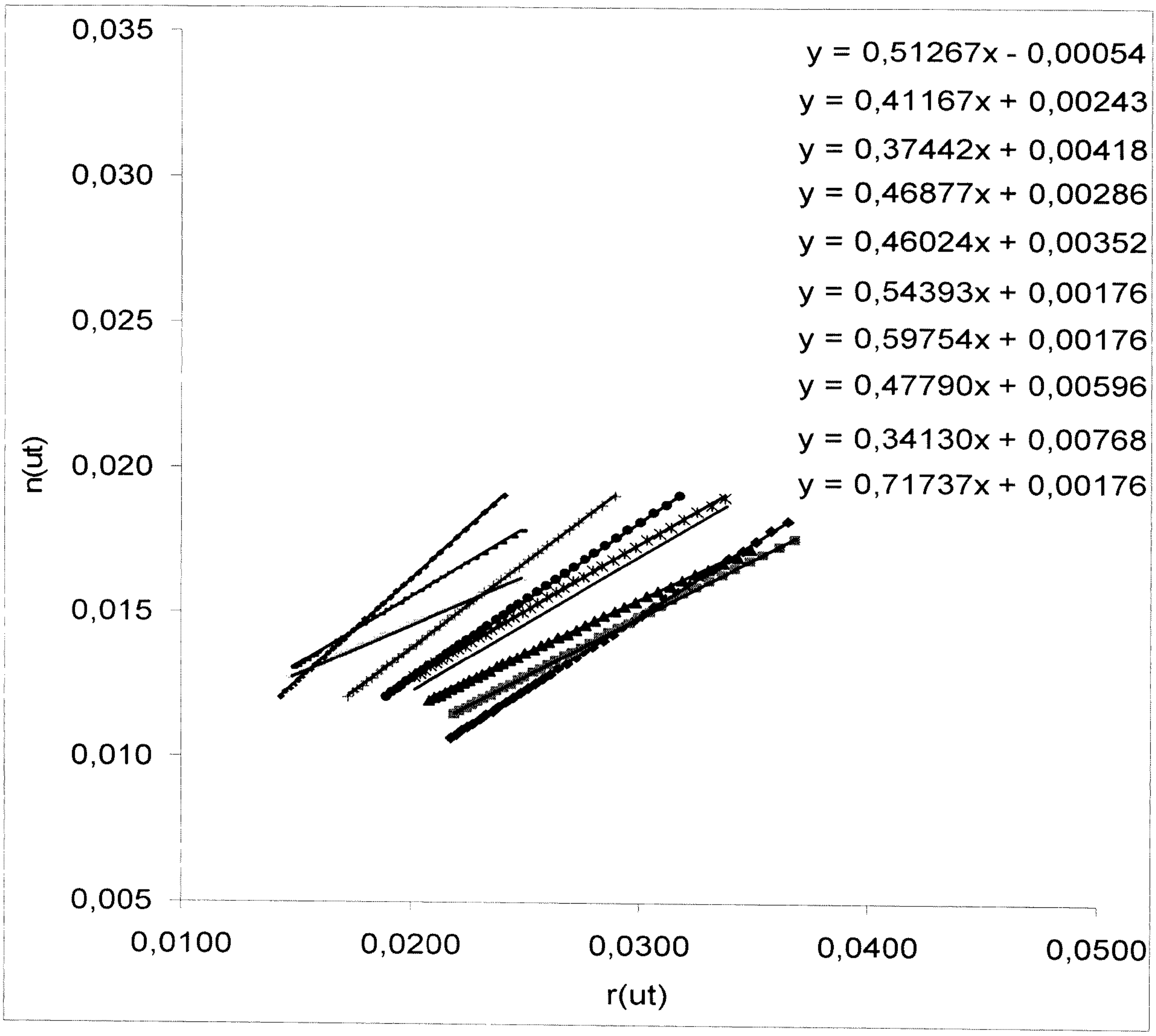
0,035
0,030
0,025
0,020
0,015
0,010
0,005
n(ut)
0,0100
0,0200
0,0300
0,0400
0,0500
r(ut)
y = 0,51267x - 0,00054
y = 0,41167x + 0,00243
y = 0,37442x + 0,00418
y = 0,46877x + 0,00286
y = 0,46024x + 0,00352
y = 0,54393x + 0,00176
y = 0,59754x + 0,00176
y = 0,47790x + 0,00596
y = 0,34130x + 0,00768
y = 0,71737x + 0,00176

FIGURE 4 :

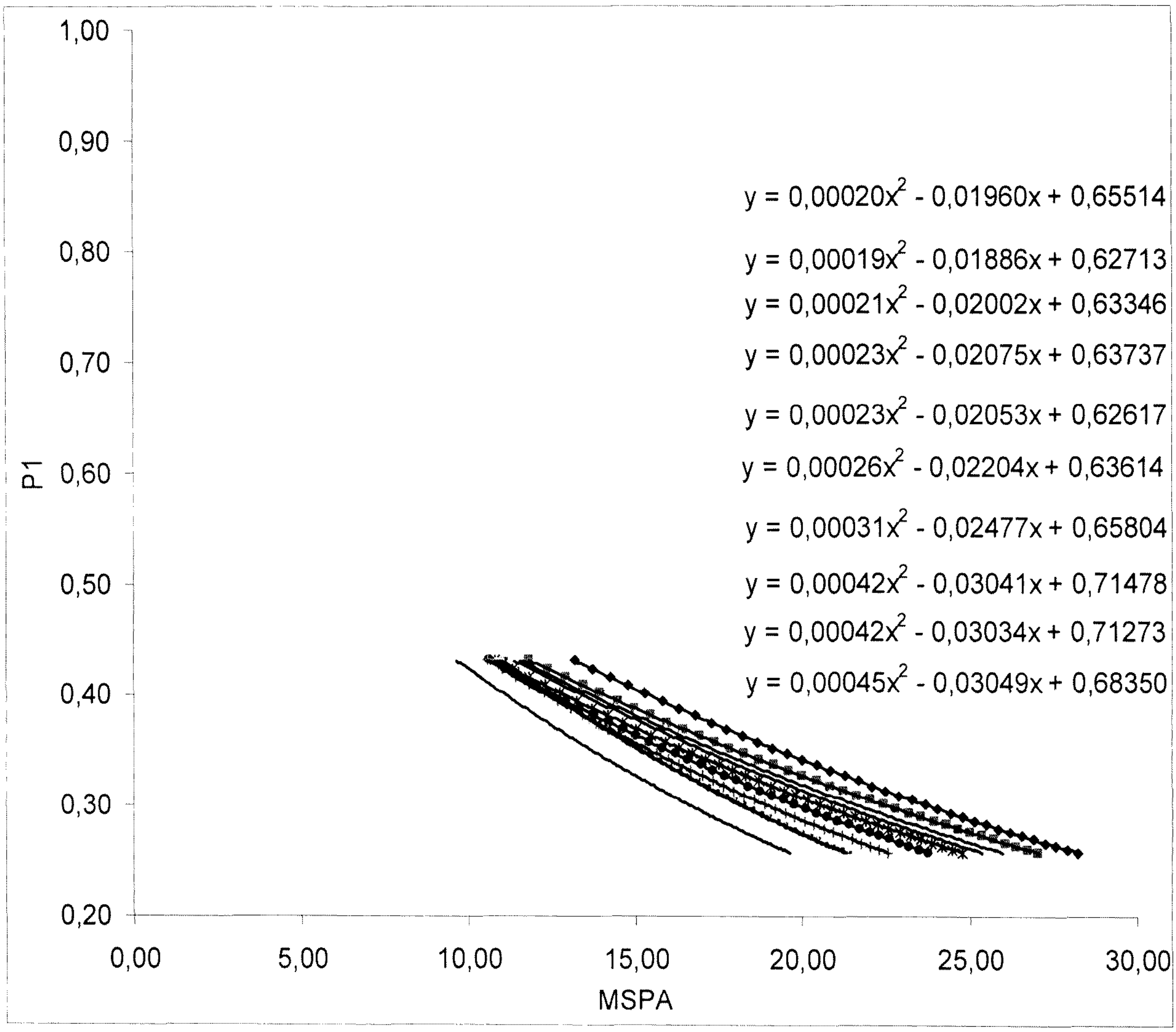
1,00
0,90
0,80
0,70
0,60
0,50
0,40
0,30
0,20
P1
y = 0,00020x2 - 0,01960x + 0,65514
y = 0,00019x2 - 0,01886x + 0,62713
y = 0,00021x2 - 0,02002x + 0,63346
y = 0,00023x2 - 0,02075x + 0,63737
y = 0,00023x2 - 0,02053x + 0,62617
y = 0,00026x2 - 0,02204x + 0,63614
y = 0,00031x2 - 0,02477x + 0,65804
y = 0,00042x2 - 0,03041x + 0,71478
y = 0,00042x2 - 0,03034x + 0,71273
y = 0,00045x2 - 0,03049x + 0,68350
0,00
5,00
10,00
15,00
20,00
25,00
30,00
MSPA

FIGURE 5 :

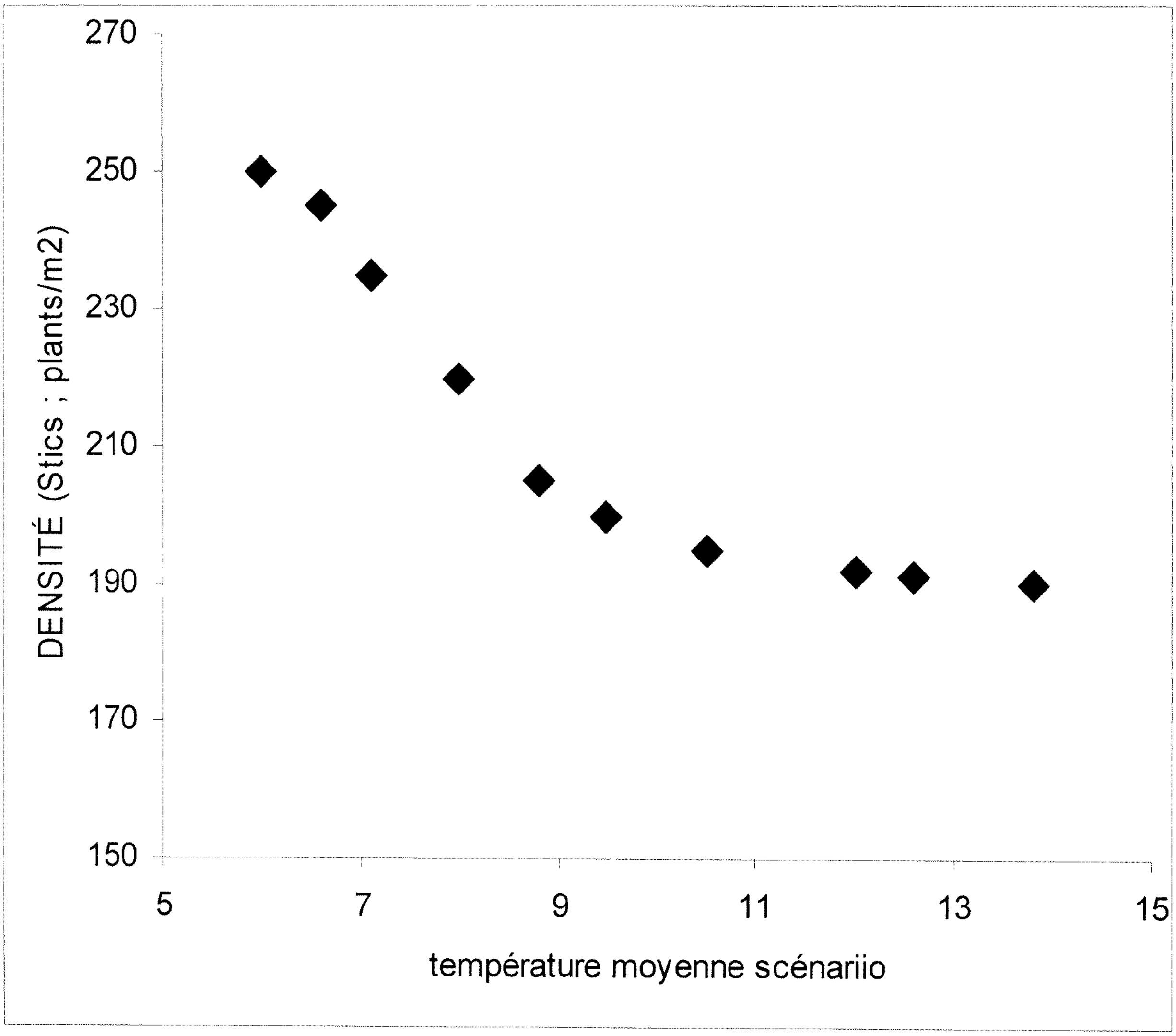
270
250
230
210
190
170
150
DENSITÉ (Stics ; plants/m2)
5
7
9
11
13
15
température moyenne scénariio

FIGURE 6 :

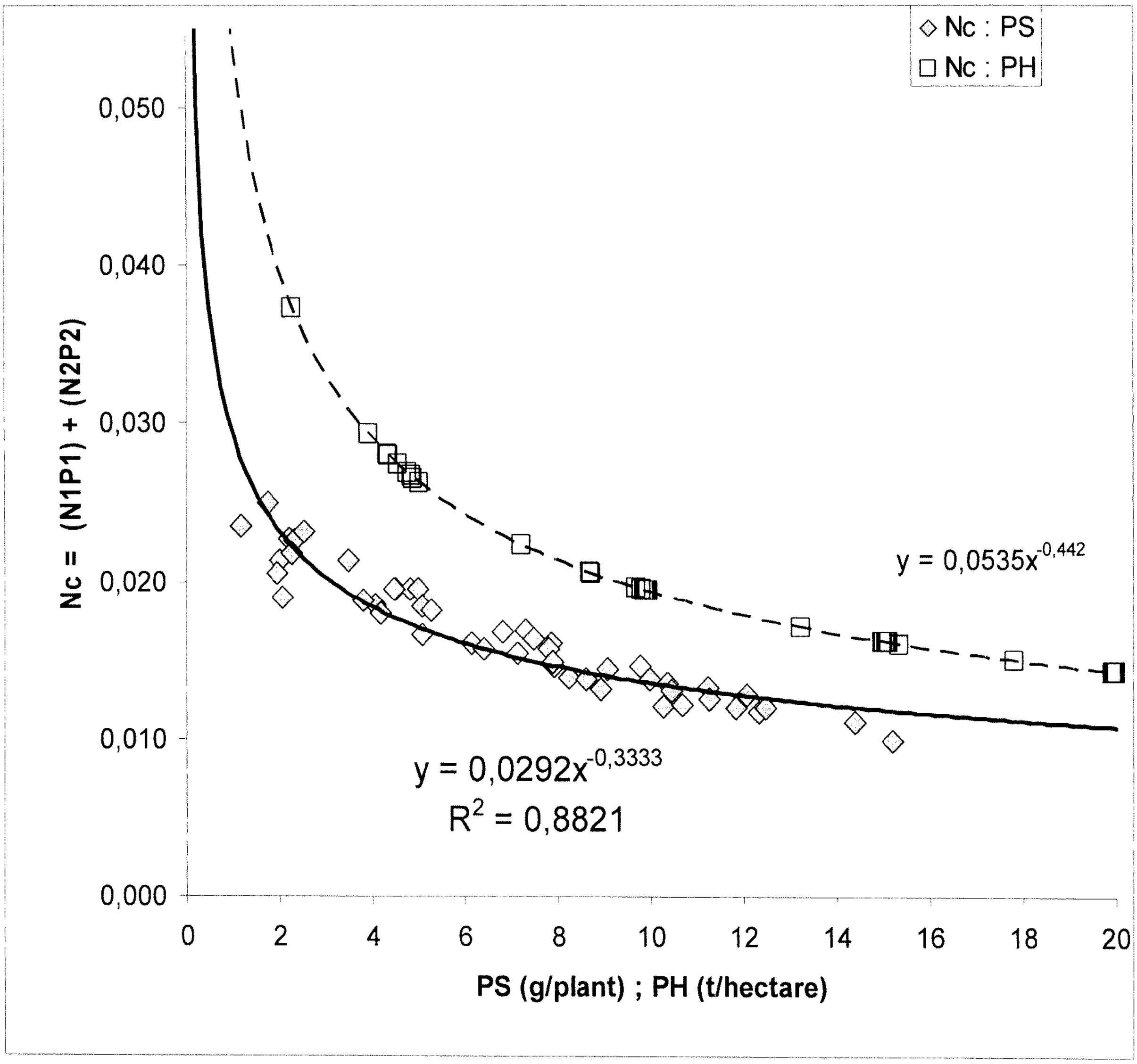
◇ Nc : PS
□ Nc : PH
0,050
0,040
0,030
0,020
0,010
0,000
Nc = (N1P1) + (N2P2)
y = 0,0535x-0,442
y = 0,0292x-0,3333
R2 = 0,8821
0
2
4
6
8
10
12
14
16
18
20
PS (g/plant) ; PH (t/hectare)

FIGURE 7

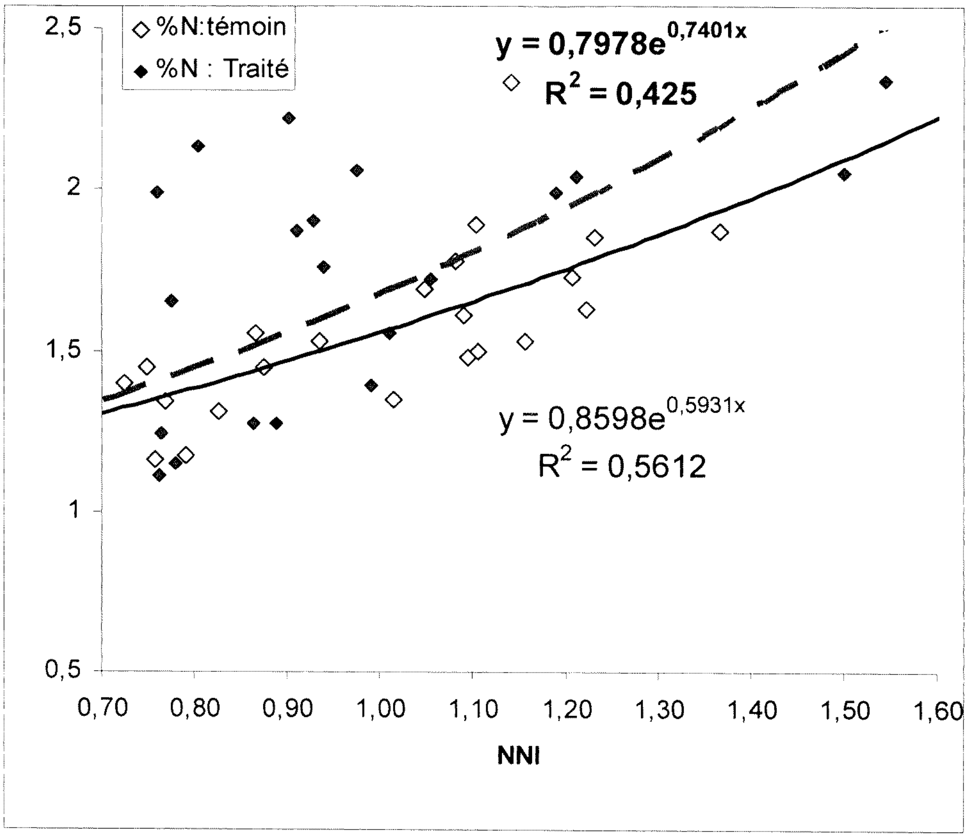
2,5
2
1,5
1
0,5
◇ %N:témoin
◆ %N : Traité
y = 0,7978e^0,7401x
R^2 = 0,425
y = 0,8598e^0,5931x
R^2 = 0,5612
0,70 0,80 0,90 1,00 1,10 1,20 1,30 1,40 1,50 1,60
NNI

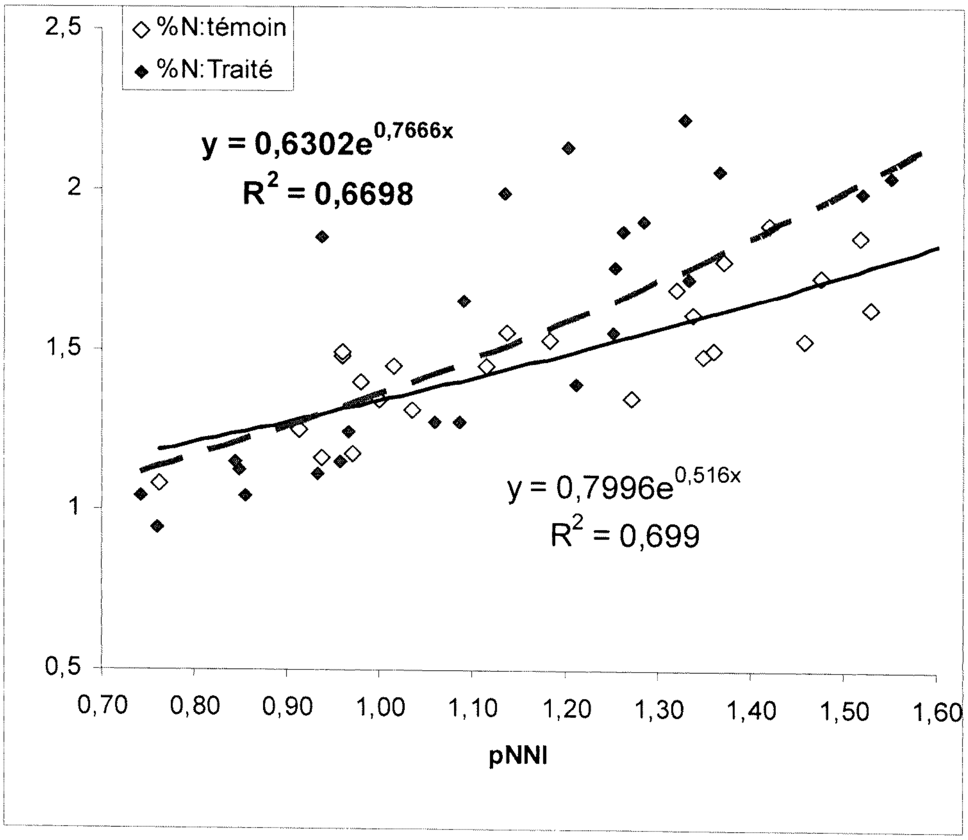
2,5
2
1,5
1
0,5
◇ %N:témoin
◆ %N:Traité
y = 0,6302e^0,7666x
R^2 = 0,6698
y = 0,7996e^0,516x
R^2 = 0,699
0,70 0,80 0,90 1,00 1,10 1,20 1,30 1,40 1,50 1,60
pNNI

FIGURE 8 :

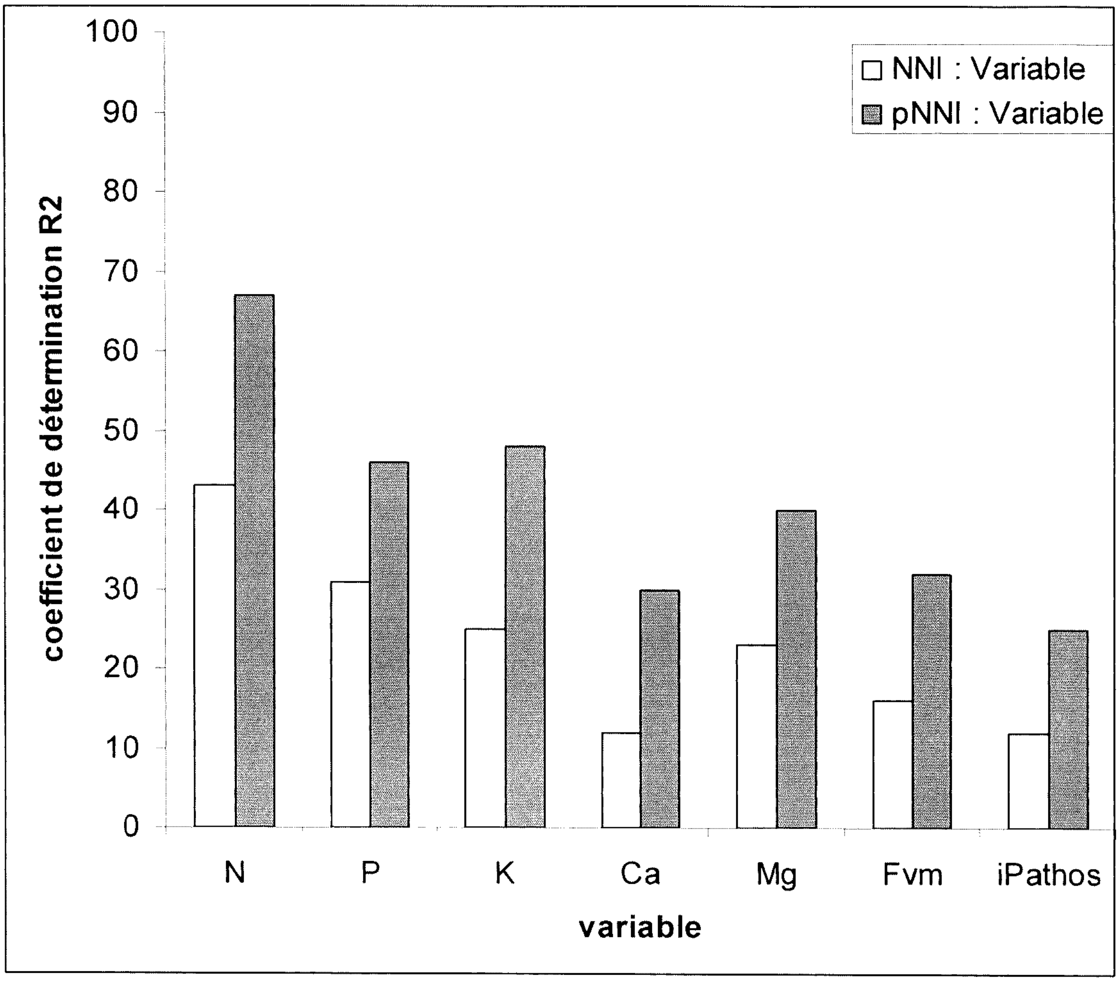
100
90
80
70
60
50
40
30
20
10
0
coefficient de détermination R2
NNI : Variable
pNNI : Variable
N
P
K
Ca
Mg
Fvm
iPathos
variable

FIGURE 9 :

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2910230
- EP 2572581 A

### Littérature non-brevet citée dans la description


- **BERMUDEZ ; MALLARINO.** CORN RESPONSE TO STARTER FERTILIZER AND TILLAGE ACROSS AND WITHIN FIELDS HAVING ,NO-TILL MANAGEMENT HISTORIES. *AGRON, J.,* 2004, vol. 96, 776-785
- **BERMUDEZ ; MALLARINO.** YIELD AND EARLY GROWTH RESPONSES TO STARTER FERTILIZER IN NO-TILL CORN ASSESSED WITH PRECISION AGRICULTURE TECHNOLOGIES. *AGRON-J.,* 2002, vol. 94, 1024-1033
- **CALOIN ; YU.** RELATION ENTRE DILUTION DE L'AZOTE ET DE CINÉTIQUE CROISSANCE CHEZ LES GRAMINÉES. *AGRONOMIE,* 1986, vol. 6 (2), 167-174
- **CALOIN ; YU.** ANALYSIS OF THE TIME COURSE OF CHANGE IN NITROGEN CONTENT IN DACTYLIS GLOMERATA L. USING A MODEL OF PLANT GROWTH. *ANNALS OF BOTANY,* 1984, vol. 54, 69-76
- **DENG et al.** *MODELS AND TESTS OF OPTIMAL DENSITY AND MAXIMAL YIELD FOR CROP PLANTS,* 2012, 15823-15828
- **DENG.** BIS. INSIGHTS INTO PLANT SIZE-DENSITY RELATIONSHIPS FROM MODELS AND AGRICULTURAL CROPS. *PNAS,* 29 Mai 2012, vol. 109 (22), 8600-8605
- **JUSTES et al.** DETERMINATION OF A CRITICAL NITROGEN DILUTION CURVE FOR WINTER WHEAT CROP. *ANNALS OF BOTANY,* 1994, vol. 74, 397-407
- **TREMBLAY, NICOLAS ; ZHIJIE WANG ; CARL BÉLEC.** PERFORMANCE OF DUALEX IN SPRING WHEAT FOR CROP NITROGEN STATUS ASSESSMENT, YIELD PREDICTION AND ESTIMATION OF SOIL NITRATE CONTENT. *JOURNAL OF PLANT NUTRITION,* 2009, vol. 33 (1), 57-70
- **WATKINSON.** YIELD-DENSITY RELATIONSHIPS: THE INFLUENCE OF RESOURCE AVAILABILITY ON GROWTH AND SELF-THINNING IN POPULATIONS OF VULPIA FASCICULATA. *ANNALS OF BOTANY,* 1984, vol. 53, 469-482
- **YAO et al.** DEVELOPMENT OF CRITICAL NITROGEN DILUTION CURVE IN RICE BASED ON LEAF DRY MATTER. *EUROPEAN JOURNAL OF AGRONOMY,* 22 Janvier 2014, vol. 55, 20-28